# EUROPEAN PATENT APPLICATION

(11) **EP 3 388 530 A1**
(43) Date of publication of application: **17.10.2018**
(21) Application number: 17166629.0
(22) Date of filing: 13.04.2017
(51) Int. Cl.: C12Q 1/68

(54) **NUCLEIC ACID MODIFICATION AND IDENTIFICATION METHOD**

(71) Applicant: IMBA-Institut für Molekulare Biotechnologie GmbH, 1030 Wien (AT)
(72) Inventor: AMERES, Stefan L., 1030 Vienna (AT); REICHHOLF, Brian, 1020 Vienna (AT); HERZOG, Veronika A., 1070 Vienna (AT)
(74) Representative: Sonn & Partner Patentanwälte

(57) **Abstract**

The invention provides a method of identifying a polynucleic acid (PNA) comprising the steps of providing a PNA; modifying one or more nucleobases of the PNA by addition or removal of a hydrogen bonding partner, thereby altering the base pairing capacity of the one or more nucleobases; base pairing a complementary nucleic acid to the PNA, including base pairing to at least one modified nucleobase; identifying the sequence of the complementary nucleic acid at least at the position that is complementary to at least one modified nucleobase.

SEQUENCE LISTING

<110>  IMBA - INSTITUT FÜR MOLEKULARE BIOTECHNOLOGIE GMBH

<120>  Nucleic acid modification and identification method

<130>  r70600

<160>  6

<170>  PatentIn version 3.5

<210>  1
<211>  76
<212>  RNA
<213>  Artificial Sequence

<220>
<223>  5L-let-7-3L

<400>  1
acacucuuuc ccuacacgac gcucuuccga ucuugaggua guagguugua uaguagaucg      60

gaagagcaca cgucuc                                                      76


<210>  2
<211>  76
<212>  RNA
<213>  Artificial Sequence

<220>
<223>  5L-let-7-s4U-p9-3L


<220>
<221>  modified_base
<222>  (42)..(42)
<223>  s4u

<400>  2
acacucuuuc ccuacacgac gcucuuccga ucuugaggua guagguugua uaguagaucg      60

gaagagcaca cgucuc                                                      76


<210>  3
<211>  34
<212>  DNA
<213>  Artificial Sequence

<220>
<223>  RT primer

<400>  3
gtgactggag ttcagacgtg tgctcttccg atct                                  34


<210>  4
<211>  58
<212>  DNA
<213>  Artificial Sequence

<220>
<223>  Solexa_PCR_fwd

<400>  4
aatgatacgg cgaccaccga gatctacact ctttccctac acgacgctct tccgatct          58


<210>  5
<211>  64
<212>  DNA
<213>  Artificial Sequence

<220>
<223>  Solexa_IDX_rev


<220>
<221>  misc_feature
<222>  (25)..(30)
<223>  n is a, c, g, or t

<400>  5
caagcagaag acggcatacg agatnnnnnn gtgactggag ttcagacgtg tgctcttccg      60

atct                                                                    64


<210>  6
<211>  21
<212>  DNA
<213>  Artificial Sequence

<220>
<223>  mature dme-let-7 sequence

<400>  6
tgaggtagta ggttgtatag t                                                 21

## Description

The present invention relates to the field of nucleic acid processing and sequencing.

### Background of the invention

Nucleotide analogs, such as 4-Thiouridine (4SU) and 6-thioguanosine (6SG) are readily incorporated into nascent RNAs, e.g. by natural enzmyes (Tani et al., Genome Res. 22, 947-956 (2012)). Among popular analogs are 5-bromouridine (5BrU), 5-ethynyluridine (5-EU), 6-thioguanosine (s⁶G) and 4-thiouridine (s⁴U), which are readily incorporated by cells and further provide unique physicochemical properties for antibody detection, cycloaddition reactions, and thiol-specific reactivity and affinity, respectively (Eidinoff et al., Science. 129, 1550-1551 (1959); Jao et al. PNAS 105, 15779-15784 (2008); Melvin et al. Eur. J. Biochem. 92, 373-379 (1978); Woodford et al. Anal. Biochem. 171, 166-172 (1988); Dölken et al. RNA 14, 1959-1972 (2008); Rabani et al. Nat Biotechnol. 29, 436-442 (2011)). 4-thiouridine (s⁴U) is the most widely used nucleotide analog to study the dynamics of RNA expression. Similar to other nucleotides, s⁴U is rapidly taken up by cells without the requirement for electroporation or lipofection. In cells, phosphorylation by cellular uridine-kinases generates an accumulating pool of phosphorylated s⁴U that is efficiently incorporated into newly synthesized RNA in a broad range of cell types including fly, murine and human cells (Dölken 2008, supra). Furthermore, cell-type-specific labeling of transcripts in vivo in flies and mice can be achieved by employing 4-thiouracil in combination with cell-type-specific expression of *Toxoplasma gondii* uracil phosphoribosyltransferase (UPRT), which couples ribose-5-phosphate to the N1 nitrogen of uracil (or 4-thiouracil) to yield (4-thio-)uridine monophosphate that is incorporated into RNA (Cleary et al. Nat Biotechnol. 23, 232-237 (2005)). Current protocols employing 4-thiouridine (s⁴U) metabolic RNA-labeling to characterize intracellular RNA biogenesis, processing, and turnover kinetics employ biochemical separation through reversible biotinylation of the thiolgroup in s⁴U [e.g. through N-[6-(Biotinamido)hexyl]-3'-(2'-pyridyldithio)propionamide (HPDP-Biotin) or biotin-coupled methanethiosulfonates (MTS-Biotin)] ( Cleary et al., 2005, supra). However, like any biochemical separation method, the underlying protocols are time-consuming and typically encounter the problem of low signal-to-noise ratios because of limitations in biotinylation efficiency (particularly when applied to short RNA species) and off-target reactivity (Duffy et al., Mol Cell. 59, 858-866 (2015); Neymotin et al., RNA 20:1645-1652 (2014)).

WO 2006/125808 A1 describes a microarray-based method of analyzing de-novo transcribed RNA that contains thiolated RNA.

WO 2004/101825 A1 and WO 2016/154040 A2 relate to methods of biosynthetic labeling and separation of RNA.

Furthermore, in reversible biotinylation strategies labeled RNA can only be analyzed in isolation, i.e. not in the context of total RNA. Precise measurements of intracellular RNA kinetics by high-throughput sequencing therefore require analysis of three RNA subsets per timepoint (labeled RNA, total RNA and unlabeled RNA), rendering these approaches expensive and downstream analyses impractical.

Therefore, it is a goal of the present invention to simplify methods of detecting modified nucleic acids, preferably to the extent to allow automated detection.

### Summary of the Invention

The present invention is based on nucleotide-analog derivatization chemistry that enables to detect modifications in polynucleotide (PNA) species at single-nucleotide resolution. The inventive method provides a scalable, highly quantitative, cost- and time-effective method for the rapid and transcriptome-wide analysis of PNA modification.

In a first aspect, the invention provides a method of identifying a polynucleic acid (PNA) comprising the steps of providing a PNA; modifying one or more nucleobases of the PNA by addition or removal of a hydrogen bonding partner, thereby altering the base pairing capacity of the one or more nucleobases; base pairing a complementary nucleic acid to the PNA, including base pairing to at least one modified nucleobase; identifying the sequence of the complementary nucleic acid at least at the position that is complementary to at least one modified nucleobase.

In preferred embodiments, the PNA is synthesized in a cell, in particular already with a modification that by itself altering the base pairing capacity or can be further modified to altering the base pairing capacity. Accordingly, the invention can also be defined as a method of identifying a polynucleic acid (PNA) comprising the steps of expressing a PNA in cell; isolating the PNA from the cell; modifying one or more nucleobases of the PNA in the cell and/or after isolation; wherein the modification(s) in the cell or after the isolation or both together add or remove a hydrogen bonding partner of one or more nucleobase, thereby altering the base pairing capacity of the one or more nucleobases; base pairing a complementary nucleic acid to the PNA, including base pairing to at least one modified nucleobase; identifying the sequence of the complementary nucleic acid at least at the position that is complementary to at least one modified nucleobase.

The invention further provides a kit for performing the inventive method, in particular a kit comprising a thiol modified nucleobase and an alkylating agent suitable for alkylating the thiol modified nucleobase at the thiol group, wherein the alkylating agent comprises a hydrogen boding donor or acceptor.

All embodiments of the invention are described together in the following detailed description and all preferred embodiments relate to all embodiments, aspects, methods and kits alike. E.g. Kits or their components can be used in or be suitable for inventive methods. Any component used in the described methods can be provided in the kit. Preferred and detailed descriptions of the inventive methods read alike on kit components or their suitability or combination of kit components for a given method step. All embodiments can be combined with each other, except where otherwise stated.

### Detailed description of the invention

The present invention relates to a method, wherein a polynucleic acid (abbreviated PNA) is modified to create synthetic PNA (also referred to as modified PNA). The presence of the synthetic PNA in a sample of PNAs can be found in the PNA sequencing readout of said sample, thereby identifying the modified PNA. An advantage of the invention is that this identification can be done without purification/separation from non-modified PNA.

In detail, the inventive method comprises the steps of modifying one or more nucleobases of a PNA by addition or removal of a hydrogen bonding partner, thereby altering the base pairing capacity (or behaviour) of the one or more nucleobases; base pairing a complementary nucleic acid to the PNA, including base pairing to at least one modified nucleobase.

Natural nucleobases are A (adenine), G (guanine), C (cytosine) and T (thymine)/U (uracil). The inventive modification leads to a nucleobase that is non-natural as compared to A, G, C, U nucleotides in case of RNA or A, G, C, T nucleotides in case of DNA. The modification leads to an altered base pairing behaviour, thereby altering the preferential base pairing (binding by hydrogen bonds) between A and T/U and between C and G. This means that the base pairing to a natural nucleobase as complementary nucleic acid changes from one natural nucleic acid to another natural nucleic acid. Preferably the complementary nucleic acid is DNA and T is used instead of U. Altered A may bind to C or G; altered T or U may bind to C or G; altered C may bind to A or T/U; altered G may bind to A or T/U. Such modifications are known in the art. Modifications are usually minor and keep changes to a minimum just so that the base pairing behaviour is changed. E.g. A and G each maintain their purine ring system and C and T/U maintain their pyrimidine ring. For example Harcourt et al. (Nature 2017, 541: 339-346) provides a review and summary of such modifications. Example modifications are modifications of A to m⁶A, to m¹A, to inosine, to 2-aminoadenine; modifications of C to m⁵C (5-methyl cytosine), to hm⁵C (5-hydroxymethyl cytosine), to pseudouridine, to 2-thiocytosine, to 5-halocytosine, to 5-propynyl (-C=C-CH₃) cytosine, 5-alkynyl cytosine; modifications of T or of U to 2-thiouracil, to s⁴U (4-thiouracil), to 2-thiothymine, to 4-pyrimidinone, to pseudouracil, to 5-halouracil, e.g. 5-bromouracil,, 5-propynyl (-C=C-CH₃) uracil, 5-alkynyl uracil, e.g. 5-ethynyluracil; modifications of G to hypoxanthine, to xanthine, to isoguanine; modifications of A or of G to 6-methyl and other 6-alkyl derivatives of adenine and guanine; to 2-propyl and other 2-alkyl derivatives of adenine and guanine. Further modifications are to 6-azo-uracil, -cytosine and -thymine, 8-halo-, 8-amino-, 8 thiol-, 8-thioalkyl-, 8-hydroxyl- and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Although the natural nucleobase is modified to preferably modified to its closest modified nucleobase as indicated above, in principle and nucleobase can be modified to any modified nucleobase as mentioned above. The relevant factor is the change in hydrogen boding pattern so that another base pairing partner will bind to the modified nucleobase as compared to the unmodified nucleobase. Change in bonding partners does not require absolute certainty, it is sufficient that a certainty of binding to a natural binding partner is changed, such as by at least 10%, at least 20%, at least 30%, at least 40%, at least 50%, at least 60%, at least 70%, at least 80%, at least 90%, or 100%. A particular nucleobase may bind more than one complementary nucleobase (especially wobble bases). Reference conditions to determine changes are at standard conditions for reverse transcriptase, preferably atmospheric pressure and 37°C, in a physiological isotonic aqueous solution. Example conditions are 50 mM Tris-HCl, 75 mM KCl, 3 mM MgCl₂, 10 mM DTT at pH 7.5-8.5. Any such change can be monitored by current detection means, such as sequencing and sequence comparison. Also, more than one modification can be included into a PNA molecule and only one per molecule or per plurality of molecules needs detection. Of course, the higher the change ratio in hydrogen bonding from one natural nucleobase to another natural nucleobase (in the complementary nucleic acid), the higher the certainty of the detection. Therefore, higher base pairing ratio changes, such as by at least 50% or at least 80%, are preferred.

"Halo" means halogen, in particular F, Cl, Br or I. "Alkyl" means an alkyl residue, preferably an alkyl residue of C₁-C₁₂ in length, branched or unbranched, substituted or not substituted. Preferred are alkyl residues of C1-C4 in length with an optional O substituent and/or an optional N substituent, such as in acetamide or any other alkyl carbonyl, carbonic acid or amide.

The base paring behaviours are known in the art or can be deduced from the changes in hydrogen bond donors or acceptors, including their obstruction to prevent their pairing. E.g. 4-pyrimidinone (modified U or T) preferably base pairs with G, instead of A (Sochacka et al., Nucleic Acids Res. 2015 Mar 11; 43 (5) : 2499-2512).

The modification of the nucleobases of the PNA can be a substitution of a hydrogen (H) on an oxygen (O) or on a nitrogen (N) atom by a substituent, such as a carbon (e.g. as in a methyl group or other alkyl group) thereby removing the H as hydrogen bond donor. The modification can be a substitution of a free electron pair of oxygen (O) or nitrogen (N) atom by substituent, such as a carbon (e.g. as in a methyl group or other alkyl group) thereby removing the electron pair as hydrogen bond acceptor. The modification may comprise the replacement of an O by sulphur (S) or SH and then performing one of the above modifications, especially alkylation of the S or SH. A preferred method of replacement of O by S or SH is by biosynthesis and providing an enzyme, e.g. a transcriptase with S or SH modified nucleotides, such as s⁴U. The transcriptase may be in a cell.

The inventive modification may be a one-step modification or a modification by more than one step, such as two, three or more steps. E.g. a first part of the modification is performed in one reaction environment, such as a cell, and a second modification is performed in another reaction environment, e.g. after isolation of the PNA from the cell. Preferably, such a second or further modification is dependent on the first modification, e.g. is performed on the atoms changed by the first modification. In particular preferred is a multi-step modification, wherein the first modification is an enzymatic modification, such as by incorporation of modified nucleotides/nucleobases by an enzyme, such as by a RNA or DNA polymerase, into the PNA. In this step, for enzymatic processivity, only small modifications are included so as not or tolerably impair enzyme activity. Small modifications are e.g. a change in only 1 or 2 atoms (not counting hydrogen) as compared to a corresponding natural nucleobase. In a further step, the incorporated modified nucleobase can be further modified by any means, e.g. to the modified nucleobases described herein, such as by wet chemical methods, including alkylation. Such a further modification can be outside a cell, enzymatic or non-enzymatic. It preferably targets the modifications introduced in the first step. A (first) modification in a cell may be an induced or enhanced modification, such by supplying the cell with modified nucleobases (e.g. as modified nucleotides), which the cell then incorporates in biosynthesised PNA. "Enhanced" means beyond natural occurrences of modifications.

It is also possible that a (first) modification is a natural process inside a cell without providing the cell with a modified nucleobase. Such a natural process is e.g. thiolation of tRNA (Thomas et al., Eur J Biochem. 1980, 113(1):67-74; Emilsson et al., Nucleic Acids Res 1992, 20(17): 4499-4505; Kramer et al., J. Bacteriol. 1988, 170(5): 2344-2351). Such naturally occurring modifications can also be detected by the inventive method, e.g. by detecting base mismatches with these modified nucleobases, or altered base pairing behaviour, directly or by a further (second) modification of these naturally modified nucleobases. Some natural modifications may be the result of a stress response or other environmental influences. Thereby, the inventive method can be used to detect such responses of a cells and influences in a cell. An example is a s4U modification, especially in tRNA, in response to UV light, especially near-UV irradiation (Kramer et al., supra). s4U modification, especially in tRNA, may also be used to measure growth rate of cells (Emilsson et al., supra). This modification, to be used as growth indicator, may be detected according to the inventive method. Preferably, eubacteria or archaea are used for such natural modification.

In preferred embodiments of the invention the step of modification is performed by incorporation of a thiol modified nucleobase into the PNA (first part of modification) and alkylating said thiol nucleobase with an alkylating agent (second part of modification). Thiol-reactive alkylating agents include iodoacetamides, maleimides, benzylic halides and bromomethylketones. Alkylating agents may comprise an alkyl group as mentioned above and a leaving group, such as halogenide, e.g. Br or Cl. The agents react by S-alkylation of thiols to generate stable thioether products. Arylating reagents such as NBD (4-nitrobenzo-2oxa-1,3-diazole) halides react with thiols or amines by a similar substitution of the aromatic halide by the nucleophile. Also available are thiosulfates for reversible thiol modification. Thiosulfates react stoichiometrically with thiols to form mixed disulfides. Thiols also react with isothiocyanates and succinimidyl esters. Isothiocyanates and succinimidyl esters may also be used to react with amines.

Modifications of a thiol may also comprise a step of converting the thiol to a thioketone. The thioketone group may then be further modified by addition or removal of a hydrogen bonding partner. The conversion to a thioketone may comprise a removal of hydrogen, such as on a transition metal cluster as catalyst as described in Köhler et al. (Angew. Chem. Int. Ed. Engl. 1996, 35(9): 993-995). The conversion to a thioketone allows additional options for reaction chemistry to perform the inventive modification. Köhler et al. also describe the introduction of a thiol or thioketone to an aryl, which is also an option for the present invention to create a thiomodification (thiol, thioketone) in the inventive modified nucleobase.

Alkylation of the thiol is also referred to as thiol(SH)-linked alkylation. The benefit of thiol alkylation is its selectivity for the "soft" thiol whereas non-thiolated nucleobases can remain unchanged. (HSAB theory - "hard and soft (Lewis) acids and bases", Pearson et al., JACS 1963, 85(22): 3533-3539). Iodoacetamides readily react with all thiols to form thioethers; they are somewhat more reactive than bromoacetamides, which may also be used. Maleimides are excellent reagents for thiol-selective modification, quantitation and analysis. In this reaction, the thiol is added across the double bond of the maleimide to yield a thioether. An alkylation is also possible via the above mentioned thioketone.

Preferably, the modification comprises alkylating on position 4 of a uridine. At this position an interference with the natural hydrogen binding behaviour of uridine is very effective. Such a modification can be with an alkylating agent, e.g. an alkylating agent that comprises the hydrogen bonding partner, preferably a hydrogen bond acceptor, or an alkylating agent that does not comprise a hydrogen binding partner - and thereby block hydrogen bonding that would normally happen at position 4 of uridine. Such an alkylation can be performed in a two-step modification via a 4-thiouridine as mentioned above.

Preferred alkylating agents have the formula Hal-(C)ₓO_{y}N_{z} (hydrogens not shown), with Hal meaning halogen, C carbon chain of x C atoms, branched or unbranched, with x being 1 to 8, O meaning y oxygen substituents to a C atom with y being 0 to 3, N meaning z nitrogen substituents to a C atom with z being 0 to 3. N is preferably at least one -NH₂ or double bonded =NH, O being preferably a -OH or double bonded =O. Hal is preferably selected from Br or I.

In particular preferred, the PNA comprises one or more 4-thiouridine, preferably 2, 3, 4, 5, 6, 7, 8, 9, 10 or more 4-thiouridine. Modifying one or more nucleobases may comprise attaching a hydrogen bonding partner, such as a hydrogen bonding acceptor or donor, to the thiol modified nucleobase. Such an attachment can be done by any chemical modification, with alkylation being preferred, such as by a halide containing alkylating agent as mentioned above.

In a preferment, a modified nucleobase, e.g. a thiol modified base, is incorporated into the PNA through biosynthesis in a cell or by cellular enzymes (e.g. by *in vitro* transcription). Also, a chemical introduction of the modified nucleobase is possible, e.g. by (non-biological) chemical PNA synthesis, such as organic or semisynthetic synthesis. Biosynthesis is the synthesis of a PNA based on a template PNA (usually DNA, in particular genomic DNA) and a template dependent synthesis (transcription, reverse transcription). Suitable enzymes for such transcription are RNA polymerases, DNA polymerases, reverse transcriptases. The enzyme can incorporate natural and modified nucleotides (with the modified nucleobase) into the biosynthesized PNA molecule. Nucleotide monomer units are connected when forming the PNA. Such monomers can be provided in modified form and incorporated into the PNA. Preferably, only one natural nucleotide type (A, G, C, T/U) is modified, i.e. has modified (non-natural) counterparts that are incorporated into the PNA. Also preferred, all natural nucleotide types are present with the modified nucleobase(s) being fewer in number than the corresponding natural (non-modified) nucleobase. "Corresponding" means the natural nucleobase with the least atom (not counting hydrogen) changes being necessary to restore the natural nucleobase. E.g. A, G, C, T/U are provided in addition modified U (or any other modified nucleotide type selected from A, G, C, T). Preferably the ratio of modified nucleotides to non-modified (natural) nucleotides of a given type is 20% or less, e.g. 15% or less or 10% or less or even 5% or less (all molar-%). The modified nucleotide will be incorporated instead of the corresponding natural nucleotide but will then later in the inventive method cause atypical base pairing (changed base pairing behaviour as detailed above), which in turn will lead to another complementary nucleotide base paring to the modified nucleotide than as it would to the natural counterpart nucleotide. Hence a change in sequence of a hybridized complementary strand, e.g. a newly synthesized complementary strand, will emerge. So, base pairing to at least one modified nucleobase may lead to base paring with another nucleotide than base pairing with a nucleobase that has not been modified, with said nucleobases being otherwise the same.

The PNA (with the modified nucleobase) may comprise or consist of RNA or DNA. Example RNA is mRNA, microRNA (miRNA or miR), short hairpin RNA (shRNA), small interfering RNA (siRNA), PIWI-interacting RNA (piRNA), ribosomal RNA (rRNA), tRNA-derived small RNA (tsRNA), transfer RNA (tRNA), small nucleolar RNA (snoRNA), small nuclear RNA (snRNA), long non-coding RNA (IncRNA), or precursor RNA molecules thereof. DNA is for example genomic DNA, cDNA, plasmidic DNA or a DNA vector. The PNA can be in a duplex or a single strand.

"Comprise" relates to an open-ended term and may also allow molecules to contain other members, e.g. other types of nucleotides (RNA or DNA, including artificially modified nucleotides such as LNA, may exist). "Consist of" is regarded as a closed definition requiring members to adhere to the requirement, i.e. complete RNA or complete DNA.

Preferably, for each nucleotide type selected from A, G, C, U or T the modified PNA comprises more natural nucleotides than modified nucleotides. Here, PNA relates to the final PNA with all modifications according to the invention. The PNA preferably comprises 1, 2, 3, 4, 5, 6, 7, 8, 9, 10 or more and up to 30 modified nucleotides. Preferably, few nucleobases are modified, such as 20% or less, e.g. 15% or less or 10% or less or even 5% or less (all molar-%) nucleobases in the PNA molecule are modified.

The PNA molecule may have any length. Preferably it has a length of at least 10 nt (nucleotides). Especially preferred is a length of 10 nt, 20 nt, 30 nt, 40 nt, 50 nt, 75 nt, 100 nt, 250 nt, 500 nt, 1000 nt, 2500 nt, 5000 nt, 10000 nt, 25000 nt, 50000 nt, 100000 nt or more nt in length or any range in between these values. Preferred ranges are of 10 nt to 100000 nt or of 50 nt to 50000 nt in length.

Preferably the PNA is from a particular cellular fraction of nucleotides, such as a total RNA fraction, a mRNA fraction or a DNA fraction, such as plasmid DNA or genomic DNA. Fractions can be selected by isolating PNA with a common characteristic, such as length, nucleotide type or sequences, such as a poly(A)-tail or a 5'-cap in mRNA.

The inventive method contains the step of base pairing the PNA by a complementary nucleic acid. In said base paring, at least one of the modified nucleobases should be base paired (usually by base pairing several nucleobases of the PNA). Base pairing with the complementary nucleic acid can be facilitated by hybridizing the PNA with a nucleic acid strand. This may also occur during extension reaction, e.g. PCR, or by hybridizing probe nucleic acids. The complementary nucleic acid may have any length, for example those lengths disclosed above for PNA.

The sequence of the complementary nucleic acid is identified at least at the position that is complementary to at least one modified nucleobase. Sequence determination can be done by any common procedure known in the art. Such methods include methods based on generating complementary strands, e.g. by PCR, completely or in part such as in next generation sequencing (NGS), a fragment based sequencing method. If desired, fragment reads can be assembled to a combined sequence. However, for the inventive uses, this not necessary as long as the complementary nucleobase to the modified nucleobase is identified, in particular with its neighbouring sequence (such as neighbours in +/-5 nt, +/-10 nt, +/-15 nt or +/-20 nt). Further methods to determine a sequence include binding to a probe, whereby through the known hybridizing probe sequence the sequence of the PNA is determined as complementary sequence.

Another option is small nucleic acid sequencing, especially if the complementary nucleic acid is small, such as in case of complementary nucleic acids to miRNA, shRNA, siRNA. Small nucleic acids may be in the length range of e.g. 10 nt to 200 nt, preferably 12 nt to 100 nt or 14 nt to 50 nt. Longer lengths than 200 nt or shorter lengths than 10 nt are also possible. The fragments of the complementary nucleic acids may have such a length on average. Fragments can be generated by physical or chemical means as known in the art for NGS. In case of small nucleic acids, including fragments such as obtained during NGS, it is preferred to ligate adaptors to the nucleic acids which may be used as hybridisation sequences for primers or probes. Such adaptors may also contain characteristic sequences, like barcodes, to identify the small nucleic acid by a label. Barcodes may provide a label for the origin of the sample from which the PNA was obtained or of the PNA molecule or its complementary nucleic acid that was fragment (fragment origin). Such barcodes may be useful in multiplexed sequencing, wherein many nucleic acids of different sequence, such a plurality of different complementary nucleic acids and/or a plurality of fragments of one or more complementary nucleic acids are sequenced. Such a plurality may e.g. be 2 to 1000 nucleic acids or more. Another possibility, that does not necessarily require adaptors, is by hybridising primers or probes to the complementary nucleic acid sequence that corresponds to the PNA. Such primers or probes may be hybridized to known sequences or randomly, e.g. by using random primers. Random primers are described below with regard to the inventive kit and any such random primer may be used in the inventive method.

In a preferred embodiment of the invention, the PNA of a single cell is identified according to the invention. Accordingly the PNA of a cell is isolated and kept separate from PNA of other cells. "Keeping PNA separate" means that the PNA of the cell under investigation remains identifiable without mixing PNA sequencing information of the cell under investigation with the sequencing information of other cells. This can be achieved by physically separating the PNA or by labelling, especially by labelling the PNA or the complementary nucleic acids with a label, e.g. by a barcode, that identifies the cell of interest. This allows the analysis of a PNA metabolism of single cells. Single cells analysis can be performed by single cell sequencing methods (Eberwine et al. Nat. Methods. 11 (1): 25-27). Alternatively to sequencing, it is also possible to prepare the complementary nucleic acids or their fragments, preferably but not necessarily with adapters, in a library. The library may then be independently sequenced or provided for other uses.

The inventive modification, such as thiol-specific alkylation, prompts the quantitative "mis-"incorporation of complementary nucleotides, which now form different hydrogen bond patterns as described above. E.g. guanosine can be incorporated instead of adenosine across the modified nucleobase (e.g. alkylated 4-thiouridine) complementary nucleic acid binding, such as during transcription or reverse transcription. Still, (reverse) transcriptase-processivity is usually unaffected since the alternatively base paired nucleotide can be amplificated together with its PNA without further hindrance. Preferred are combinations with a second modification after a first enzymatic modification (such as by incorporating modified nucleobases). Such a combination with well-established and non-toxic s⁴U metabolic labeling protocols as mentioned above.

The inventive sequencing method that leads to a sequence change in the complementary nucleic acid due to the modified nucleobase can be coupled to available high-throughput sequencing methods, such as NGS. Sequence changes, in particular if incomplete or partial, differing between different individual molecules of PNA/complementary nucleic acids can be identified by available computational methods. E.g T>C conversions (due to U modifications that lead to increased G base pairing) can be tracked in next-generation sequencing datasets. Such highly automated methods, in combination with computerized analysis, allows the invention to provide rapid access to intracellular RNA processing kinetics, a preferred application of the invention. The invention can accurately report the RNA polymerase II-dependent transcriptional output due to complementary base pairing. Insights into the intracellular kinetics of RNA biogenesis, processing and turnover is essential to unravel the molecular basis for changes in gene expression patterns that impinge on essentially any given biological process in life.

Accordingly, in a preferment, the inventive method can be used to determine modifications or easily modified alterations of PNAs in cells. Such "easily modified alterations" e.g. relate to the above described multi-step method, wherein a first modification (also termed alteration) is performed in a cell and a later modification is done in a second or further step, usually outside a cell after isolation of the PNA.

Preferably, the inventive method is used to modify RNA (as PNA) with at least a first modification/alteration performed in a cell, in particular in living cell. This allows tracking of RNA expression changes since expressed RNA is modified.

The regulated expression of genetic information is essential to maintain cellular homeostasis, provides cellular flexibility to respond to altering environmental conditions, and - if dysregulated - contributes to human diseases such as cancer. Underlying these essential biological processes are tightly regulated molecular events that control the relative kinetics of RNA transcription, processing, and degradation in a transcript-specific manner.

The cellular RNA pool, encompassing a myriad of RNA species - including mRNA or non-coding RNAs, such as microRNAs - is defined by the transcription of selected loci in the genome, and can be qualitatively and quantitatively assessed by RNA profiling techniques, such as high-throughput sequencing. However, the abundance measurement of steady-state RNA levels does not accurately mirror transcriptional activity per se. In fact, RNA stability plays a major role in determining the relative abundance of any given RNA molecule. Approaches to measure transcription and RNA decay rates at the genomic scale are therefore useful to unravel insights into the dynamics of RNA expression and its underlying regulatory mechanisms. According to the invention, it is possible to determine the intracellular kinetics of RNA biogenesis and turnover.

RNA can be altered or modified by a cells own metabolism, e.g. by incorporating altered or modified nucleotides into naturally processed RNA. Such alterations can be used to selectively introduce the inventive modifications that (alone or after a further modification) change hydrogen bonding behaviour. Due to the metabolic influence, such a method is referred to as "metabolic sequencing" - in case the modified nucleotide is then sequenced. The sequencing step or generally any base pairing step to a complementary (poly)nucleotide can be automated and processed in a high-throughput sequencing method as mentioned above. The invention provides a high-throughput-compatible metabolic labelling protocol that is suitable to determine the intracellular kinetics of RNA biogenesis and turnover. It accurately measures RNA polymerase II-dependent poly-adenylated transcriptional output, and recapitulates global post-transcriptional gene regulatory signatures, thus solving the problem of providing RNA expression kinetics (including biogenesis and turnover) in a cell at high temporal resolution.

The cell can be any cell, such as a bacterial cell, including eukaryotic and procariotic cells, gram negative and gram positive cells, fungal cells, algae cells, plant cells, animal cells, mammalian cells, such as rodent cells, primate cells, human cells, non-human cells, archaebacterial cells, avian cells, amphibian cells, such as frog cells, reptilian cells, marsupial cells.

It is possible to monitor changes by temporal control of the modification, e.g. a phase of cell RNA expression without modification is compared with phase of cell RNA expression with modification. Such phases are preferably compared in the same cell or cell culture. E.g. a phase with modification is followed by a phase without modification or vice versa. Accordingly in a preferment of the invention, one or more cells are cultured in at least two culturing phases, wherein one culturing phase comprises incorporation of a modified nucleotide into biosynthesized RNA, which is modified by addition or removal of a hydrogen bonding partner; and another culturing phase that lacks such incorporation of the modified nucleotide into biosynthesized RNA. It is also possible that the "another culturing phase" comprises incorporation of a modified nucleotide into biosynthesized RNA but at a different, e.g. lower, concentration as in the other one culturing phase. The different or lower concentration as in the other one phase should be sufficient to observe a difference (in particular different concentration) in the incorporation of modified nucleotides into biosynthesized RNA. The inventive method can be accordingly defined as a method of identifying a polynucleic acid (PNA) comprising the steps of expressing a PNA in cell; modifying one or more nucleobases of the PNA; isolating the PNA from the cell; optionally further modifying the PNA; wherein the modification(s) before or after the isolation or together add or remove a hydrogen bonding partner of one or more nucleobase, thereby altering the base pairing capacity of the one or more nucleobases; base pairing a complementary nucleic acid to the PNA, including base pairing to at least one modified nucleobase; identifying the sequence of the complementary nucleic acid at least at the position that is complementary to at least one modified nucleobase. A particular preferred metabolic-labeling (i.e. modification by a cells metabolism, such as by its enzymes like RNA polymerases) is by 4-thiouridine-incorporation events. This can be used to change the base pairing behavior of U.

In particular preferred is the method in at least two culturing phases of cells, wherein in at least two culturing phases different levels of PNA modification, in particular RNA modification, are facilitated. This can be achieved by providing the cell with different concentrations of the modified nucleobase, thereby allowing the cell to incorporate the modified nucleobase at different levels or concentrations into PNA, especially RNA. As above, preferably the modified nucleobase is a thiol modified nucleobase. The level of PNA modification in one phase may be no modification. The phases, especially those with PNA modification should have a pre-set time period for said PNA modification. By comparing the incorporation between the different phases it is possible to calculate a turnover rate in the pre-set time period. In a particular preferred embodiment, a turnover or degradation rate is calculated based on the incorporation of modified nucleobase into PNA in at least one phase in comparison to the other phase. Preferably, the phases are consecutive cultivation phases.

A further comparison can be between cultivation phases of different cells. Such a comparison allows an estimate of differential expression and PNA turnover between these cells. One of the cells or group of cells may be a control and another cell or another group of cells may be candidate cell or group of cells under investigation. Both cells or group of cells may have a phase of incorporation of modified nucleobases into PNA, which is compared. Preferably such a phase of incorporation is controlled by providing the cells with modified nucleobases for incorporation into PNA. Preferably the same amounts of modified nucleobases are provided to each cell or to each group of cells, suitable for comparison of cell metabolism. Preferably a phase of incorporation is followed by a phase of no further incorporation, e.g. by ceasing to supply the cell or group of cells with further modified nucleobases. It is also possible that a phase of incorporation is followed by a phase of reduced incorporation or by incorporation at different levels. Any change in levels of incorporation of modified nucleobases into PNA is followed by an adaption of the cell's metabolism, which may be monitored by the inventive method. E.g. if a phase of incorporation is followed by a phase of lower or no incorporation, then it is possible to monitor degradation of modified PNA. If a phase of no or limited incorporation is followed by a phase of incorporation or higher then limited incorporation, then it is possible to monitor build-up of modified PNA.

The cell or group of cells may be in a culture *in vitro* or in a living organism, such as a plant, bacterial cell, fungal cell, algae cell, non-human animal or human, *in vivo.* It case of *in vivo* cells, the modified nucleobases may be supplied to the cell by administering the modified nucleobases to the organism, e.g. systemically like into a vascular system or topically to an organ of interest of the organism. Accordingly, it is possible to monitor metabolism of PNA *in vivo,* or in a particular organ of interest. The PNA may then be isolated from the organism, such as by a biopsy or, in case of secreted PNA, from a body fluid sample, or by sacrificing a non-human organism. Preferably PNA of single cells from the organism is isolated and analyzed according to the inventive method, e.g. by labelling and/or library generation and/or by single cell sequencing as mentioned above. Any description of culturing phases also applies to treatment *in vivo* and is referred to as "growth phase". "Growth phases" do not require growth of cells or multiplication of cells but refer to the PNA metabolism or "growth" that is identified and analyzed.

Comparison of different levels of PNAs and PNA turnover is important to elucidate differences in cell metabolism between different states cells are in during an organism's development and disease. To be able to measure turn overrate of PNAs can help elucidate which pathways are active and which are less active or inactive. In that respect the turnover rate provides for an additional measure to the steady state concentration measurement of PNA, in particular RNA, measuring just the concentration of PNA, such as mRNA present in a cell or tissue or organ.

Preferably the biosynthesized PNA, preferably RNA, of the two culturing phases are collected from said cells, preferably also mixed, and wherein base pairing a complementary nucleic acid to the PNA comprises generation of complementary polynucleic acid strands, preferably DNA strands, by transcription, such as reverse transcription in case of RNA as PNAs.

It is a particular benefit of the invention that the PNA created with modification and comparable PNA without or less modification, or the respective complementary nucleic acids need no separation. The base pairing of the PNA with the complementary nucleic acids can be in mixture - of both modified PNA and non-modified PNA. The sequence of the PNA/complementary nucleic acids can then be determined in combination because the sequence/identity of the complementary nucleic acids can be determined in both cases (with and without modification) and by comparison the modification events can be inferred. Such comparison is preferably a computerized sequence comparison. The inventive method, especially preferred according to its embodiment of base pairing to at least one modified nucleobase that leads to base paring with another nucleotide than base pairing with a nucleobase that has not been modified, further comprises determining the sequence of the complementary polynucleic acid strands and comparing the strand sequences, wherein an altered complementary nucleic acid as a result of the modification by addition or removal of a hydrogen bonding partner can be identified by comparison with the complementary nucleic acid without modification. Preferably the sequences of nucleotides are determined as fragments, such as used in NGS and high throughput sequencing. Sequences to be determined (which many harbour the position that is complementary to the at least one modified nucleobase) may have a length of 10 nt to 500 nt, preferably of 12 nt to 250 nt or of 15 nt to 100 nt.

Computerized identification of the sequence of the complementary nucleic acid at least at the position that is complementary to at least one modified nucleobase may comprise a comparison with a sequence of a non-modified PNA. Such comparative sequences may be obtained from sequence databases such as at EBI or at NCBI or determined by PNA generation without introducing a modification, such as by natural bases base pairing to natural complementary bases. A computer program product for such comparison or a computer readable medium for the method can be included in the inventive kit.

The invention further provides a kit suitable for performing a method of the invention comprising a thiol modified nucleobase and an alkylating agent suitable for alkylating the thiol modified nucleobase at the thiol group, wherein the alkylating agent comprises a hydrogen bonding donor or acceptor, preferably wherein the alkylating agent is any one mentioned above, especially preferred iodoacetaminde. However, any of the above described alkylating agents, agents suitable for any of the above modifications, in particular modified nucleotides with the modified base, such as thiol modified nucleotides can be included in the inventive kit.

The kit preferably further comprises primers, nucleotides selected from A, G, C, and T, a reverse transcriptase or a combination thereof, preferably all these components. Example primers are random primers, which are mixtures of randomly selected primers. Such a random primer mixture may have at least 50 or at least 100, at least 500 different primers. Random primer may contain random hexamers, random pentamers, random pentamers random octamers, etc.

The kit may further comprise a PNA polymerase and preferably further a buffer for polymerization of the polymerase. The polymerase may be DNA or RNA polymerase.

The inventive kit may also comprise adaptor nucleic acids. Such adaptors may be ligated to nucleic acids to generate adaptor bound complementary nucleic acids as described above. The adaptors may comprise one or more barcodes as described above. The kit may also comprise a ligase, such as a DNA ligase.

The components of the kit may be provided in suitable containers, such as vials or flasks.

The kit may also comprise instructions or a manual for performing any of the inventive method.

The present invention is further described by the following figures and examples, without necessarily being limited to these aspects of the invention.

### Figures:

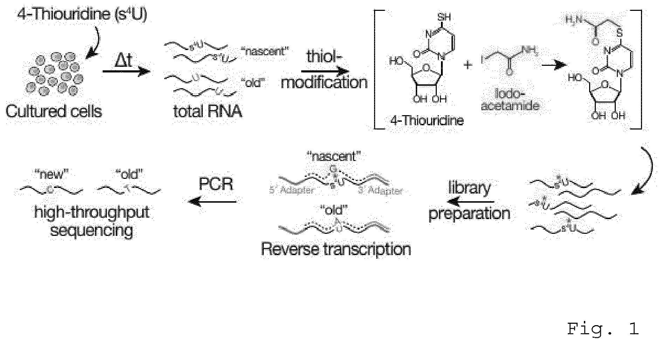

**Figure 1****. Schematic overview of thiol(SH)-linked alkylation for the metabolic sequencing of RNA.** Cells are treated with 4-thiouridine (s⁴U), which upon cellular uptake incorporates into newly transcribed RNA. Upon total RNA preparation at given time points s⁴U-residues present in newly generated RNA species are carboxyamidomethylated by treatment with iodoacetamide (IAA), resulting in a bulky group at the base-pairing interface. When combined with well-established RNA library preparation protocols, the presence of the bulky group at the sites of s⁴U - incorporation leads to the specific and quantitative misincorporation of G across alkylated s⁴U during reverse transcription (RT). s⁴U-containing sites can be identified bioinformatically in high-throughput sequencing libraries at single nucleotide resolution by calling T-to-C transitions.

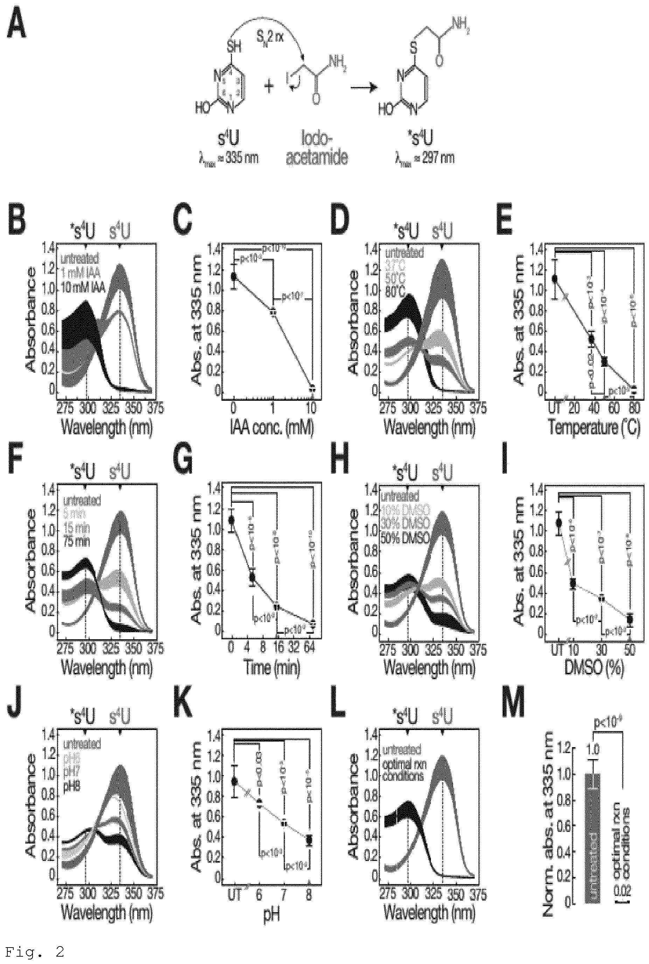

**Figure 2****. 4-thiouracil-derivatization by thiol-linked alkylation. (A)** 4-thiouracil (s⁴U) reacts with the thiol-reactive compound iodoacetamide (IAA), attaching a carboxyamidomethyl-group to the thiol-group in s⁴U as a result of a nucleophilic substitution (S_{N}2) reaction. Absorption maxima of educt (4-thiouracil; s⁴U; λₘₐₓ ≈ 335 nm) and product (carboxyamidomethylated 4-thiouracil; *s⁴U; λₘₐₓ ≈ 297 nm) are indicated. **(B)** Absorption spectra of 4-thiouracil (s⁴U) in the absence and presence of the indicated concentration of iodoacetamide (IAA). 1 mM s⁴U was incubated with the indicated concentration of IAA for 1h at 37°C in the presence of 50 mM sodium phosphate buffer (pH 8.0) and 10% DMSO. Data represents mean ± SD of at least three independent replicates. **(C)** Quantification of absorption at 335 nm as shown in (B). P-values (Student's t-test) are indicated. **(D)** Absorption spectra of 1 mM 4-thiouracil (s⁴U) in the absence and presence of 10 mM iodoacetamide (IAA) after incubation at the indicated temperature for 5min in the presence of 50 mM sodium phosphate buffer (pH 8.0) and 10% DMSO. Data represents mean ± SD of at least three independent replicates. **(E)** Quantification of absorption at 335 nm as shown in (D). P-values (Student's t-test) are indicated. **(F)** Absorption spectra of 1 mM 4-thiouracil (s⁴U) in the absence and presence of 10 mM iodoacetamide (IAA) after incubation at 37°C for the indicated time in the presence of 50 mM sodium phosphate buffer (pH 8.0) and 10% DMSO. Data represents mean ± SD of at least three independent replicates. **(G)** Quantification of absorption at 335 nm as shown in (F). P-values (Student's t-test) are indicated. **(H)** Absorption spectra of 1 mM 4-thiouracil (s⁴U) in the absence and presence of 10 mM iodoacetamide (IAA) after incubation at 50°C for 2min in the presence of 50 mM sodium phosphate buffer (pH 8.0) and the indicated amount of DMSO. Data represents mean ± SD of at least three independent replicates. **(I)** Quantification of absorption at 335 nm as shown in (H). P-values (Student's t-test) are indicated. **(J)** Absorption spectra of 1 mM 4-thiouracil (s⁴U) in the absence and presence of 10 mM iodoacetamide (IAA) after incubation at 50°C for 5min in the presence of 50 mM sodium phosphate buffer with the indicated pH and the 10 % DMSO. Data represents mean ± SD of at least three independent replicates. **(K)** Quantification of absorption at 335 nm as shown in (J). P-values (Student's t-test) are indicated. **(L)** Absorption spectra of 1 mM 4-thiouracil (s⁴U) in the absence and presence of 10 mM iodoacetamide (IAA) after incubation at 50°C for 15min in the presence of 50 mM sodium phosphate buffer (pH 8.0) and 50 % DMSO (optimal reaction [rxn] conditions). Data represent mean ± SD of at least three independent replicates. **(M)** Quantification of absorption at 335 nm as shown in (J). P-values (Student's t-test) are indicated.

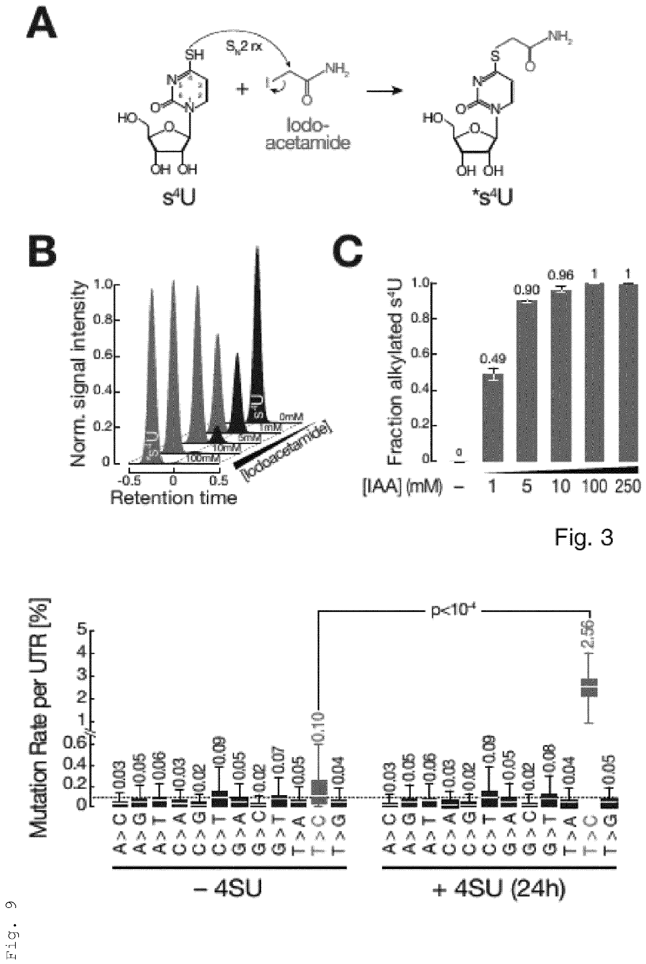

**Figure 3****. 4-thiouridine-derivatization by thiol-linked alkylation. (A)** 4-thiouridine (s⁴U) reacts with the thiol-reactive compound iodoacetamide (IAA), attaching a carboxyamidomethyl-group to the thiol-group in s⁴U as a result of a nucleophilic substitution (S_{N}2) reaction. **(B)** Analysis of s⁴U-alkylation by mass spectrometry. 40 nmol 4-thiouridine were incubated with the indicated concentration of iodoacetamide in standard reaction buffer (50 mM NaPO4 (pH 8), 50 % DMSO) at 50°C for 15min. The reaction was stopped with 1% acetic acid. Acidified samples were separated on a Ulitimate U300 BioRSLC HPLC system (Dionex; Thermo Fisher Scientific), employing a Kinetex F5 Pentafluorophenyl column (150 mm x 2.1 mm; 2.6 µm, 100 Å; Phenomenex) with a flow rate of 100 µl/min. Nucleosides were on-line analyzed using a TSQ Quantiva mass spectrometer (Thermo Fisher Scientific) after electrospray ionization with the following SRMs: 4-Thiouridine m/z 260 → 129, alkylated 4-Thiouridine m/z 318 → 186. Data were interpreted using the Trace Finder software suite (Thermo Fisher Scientific) and manually validated. **(C)** Quantification of two independent experiments in two technical replicates shown in (B). Fraction alkylated s⁴U at indicated IAA concentrations represent relative normalized signal intensities at peak retention times of s⁴U and alkylated s⁴U. Data represent mean ± SD.

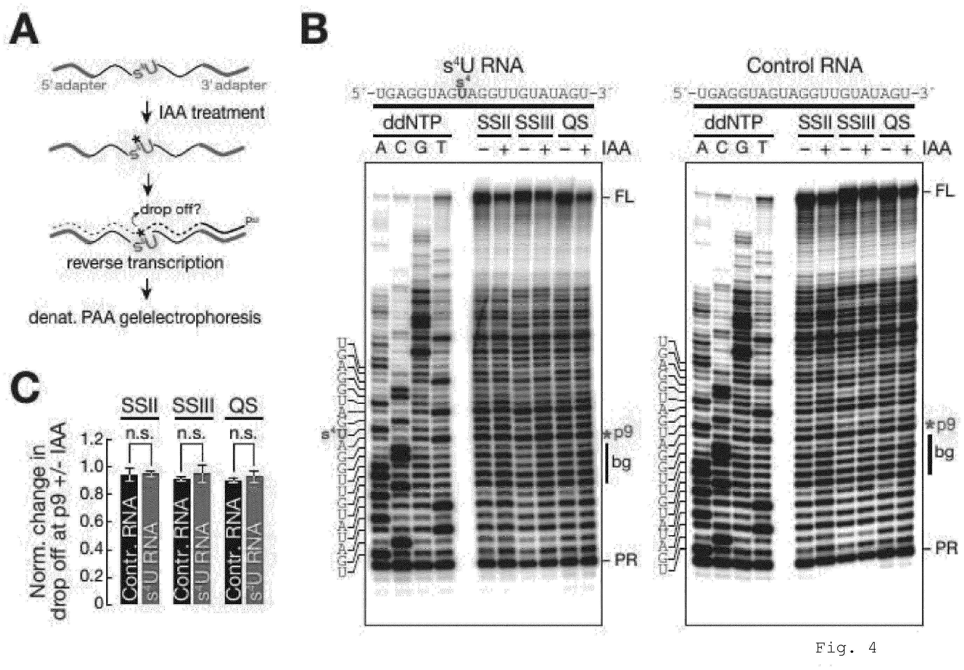

**Figure 4****. Alkylation of 4-thiouridine-containing RNA does not affect reverse transcription processivity. (A)** To determine the effect of s⁴U-alkylation on reverse transcriptase-processivity we employed a synthetic 76 nt long RNA that contains 4-thiouridine (s⁴U) incorporation at a single position (p9) within the sequence of the *Drosophila* small RNA *dme-let-7*, flanked by 5' and 3' adapter sequences. Reverse transcription was assayed before and after treatment with iodoacetamide (IAA) using commercially available reverse transcriptases by following the extension of a 5' ³²P -labeled DNA oligonucleotide, reverse and complement in sequence to the 3' adapter sequence. **(B)** Reactions as prepared in (A) were analyzed by polyacrylamide gel electrophoresis followed by phosphorimaging. Primer extension results of s⁴U-containing and non-containing RNA in the presence and absence of IAA-treatment, conducted with the reverse transcriptases Superscript II (SSII), Superscript III (SSIII) or Quant-seq RT (QS) are depicted. The sequence of the RNA component excluding adapter sequences are shown; the position of the s⁴U residue is indicated in red. RNA sequencing was performed by addition of the indicated ddNTPs to the reverse transcription reaction. PR, 5' ³²P -labeled DNA primer; bg, background stop signal; *p9, termination signal at position 9; FL, full length product. **(C)** Quantification of three independent replicates of experiment shown in (B). Ratio of drop off signal (+ vs - IAA treatment) at p9 after normalization to preceding background drop off signal was determined for control and s⁴U-containing RNA employing the indicated reverse transcriptases. Data represent mean ± SD. Statistical analysis was performed using Student's t-test.

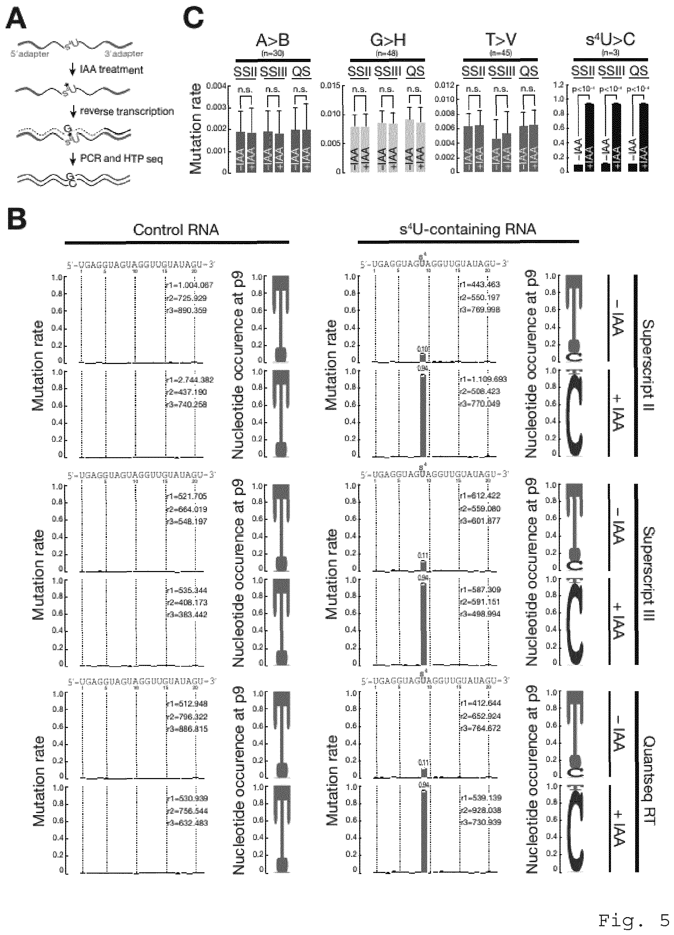

**Figure 5****. Alkylation enables the quantitative identification of s⁴U-incorporations in RNA at single nucleotide resolution. (A)** RNA with or without 4-thiouridine (s⁴U) incorporation at a single position (p9) was treated with iodoacetamide (IAA) and subjected to reverse transcription and gel-extraction of full-length product followed by PCR amplification and high-throughput (HTP) sequencing. **(B)** Mutation rates for each position of a control RNA (left panels) and a s⁴U-containing RNA (right panels) in the presence or absence of iodoacetamide (IAA) treatment employing the indicated reverse transcriptase are shown. Bars represent average mutation rates ± SD of three independent replicates. Numbers of sequenced reads in each replicate (r1-r3) are indicated. Nucleotide identity occurrence at p9 is shown. **(C)** Mutation rates for the indicated mutations in the presence or absence of iodoacetamide (IAA) treatment employing Superscript II (SSII), Superscript III (SSIII), or Quant-seq reverse transcriptase (QS). Mutation rates were averaged across positions with the same nucleotide identity for both, s⁴U-containing and non-containing RNA oligonucleotides. P-Values (determined by Student's t-test) are indicated. N.s., not significant (p>0.05).

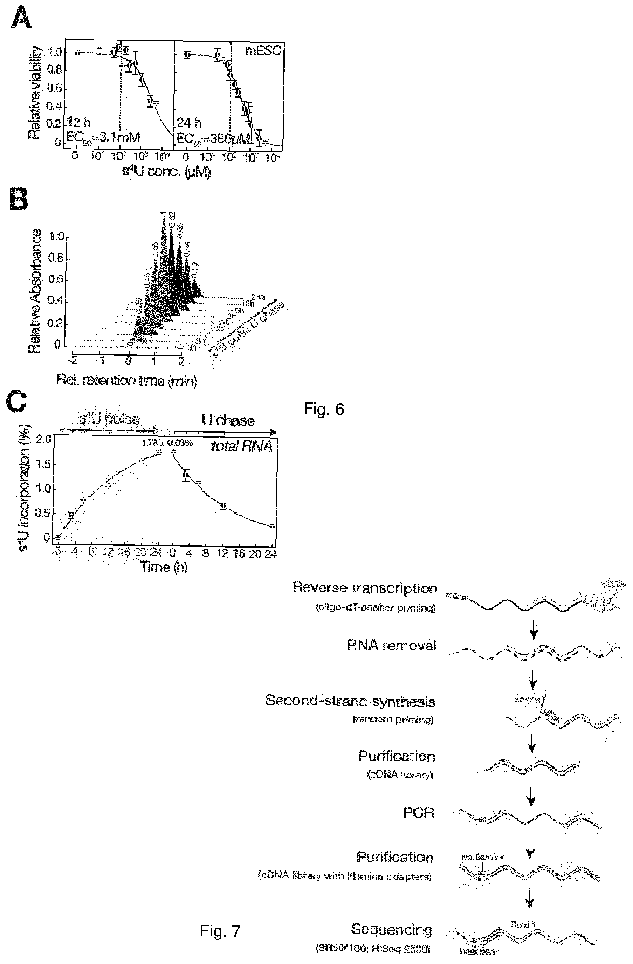

**Figure 6****. Effect of s⁴U treatment on mES cell viability and metabolic RNA labeling. (A)** Viability of mES cells cultured in the presence of the indicated concentration of 4-thiouridine (s⁴U) for 12h (left) or 24h (right) relative to untreated conditions is shown. Final concentration used in subsequent experiments (100 µM) is indicated by triangle and dotted line. **(B)** Quantification of s⁴U-incorporation into total RNA after s⁴U-metabolic labeling for the indicated time in a pulse, or following media replacement in a uridine chase. s⁴U-incorporation was determined by HPLC analysis following digestion and dephosphorylation of total RNA to single nucleosides. Background-subtracted s⁴U signal intensities at 330 nm normalized to 24h pulse labeling timepoint and absorbance of uridine signal intensities at 260 nm is shown. Column retention time (min) relative to s⁴U-adsorption maxima is shown. **(C)** Substitution rate of s⁴U compared to unmodified uridine determined by HPLC. s⁴U incorporation in total RNA across all timepoints of a s⁴U-metablic pulse and chase labeling experiment in mES cells. Values represent mean±SD of three independent replicates. Maximum incorporation rates after 24h labeling are indicated.
**Figure 7****. Quant-seq mRNA 3' end sequencing library preparation protocol.** Quant-seq uses total RNA as input, hence no prior poly(A) enrichment or rRNA depletion is required. Library generation is initiated by oligo(dT) priming. The primer already contains Illumina-compatible linker sequences (shown in green, top: "adapter", next step: last bend). After first strand synthesis the RNA is removed and second strand synthesis is initiated by random priming and a DNA polymerase. The random primer also contains Illumina-compatible linker sequences (shown in blue). No purification is required between first and second strand synthesis. The insert size is optimized for shorter reads (SR50 or SR100). Second strand synthesis is followed by a magnetic bead-based purification step. The library is then amplified, introducing the sequences required for cluster generation (shown in red and purple). External barcodes (BC) are introduced during the PCR amplification step for multiplexing.

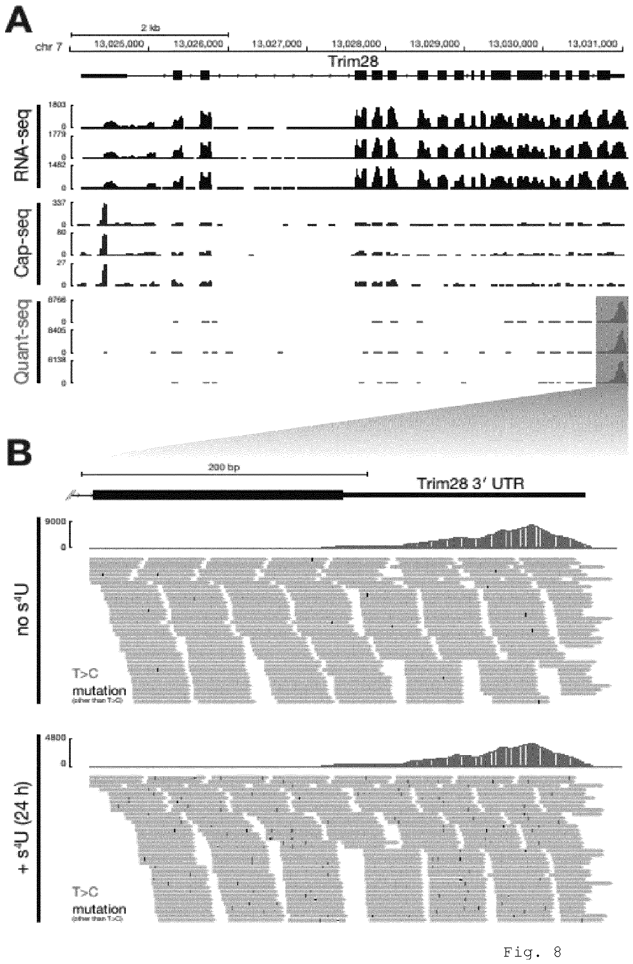

**Figure 8****. s⁴U incorporation events in mRNA of mES cells upon metabolic labeling. (A)** Representative genome browser screen shot for three independent mRNA libraries generated from total RNA of mES cells, prepared using standard mRNA sequencing (top three panels), Cap analysis gene expression (CAGE; middle three panels) and mRNA 3' end sequencing (bottom three panels). A representative area in the mouse genome encoding the gene Trim 28 is shown. **(B)** Zoom into the genome area encoding the mRNA 3' end of Trim28 including its 3' untranslated region (UTR). Coverage plots of mRNA 3' end sequencing libraries prepared from total RNA of untreated mES cells or mES cells subjected to s⁴U-metabolic labeling for 24h followed by modification and seqeuncing are shown. A subset of individual reads underlying the coverage plots is depicted. Red bars within individual reads represent T>C conversions; black bars represent any mutation other than T>C.
**Figure 9. Global analysis of mutation rates in mRNA 3' end sequencing following s⁴U metabolic RNA labeling in mES cells.** mRNA 3' end sequencing libraries generated from total RNA of mES cells before and after s⁴U metabolic labeling for 24h were mapped to annotated 3' untranslated regions (UTRs) and any given mutation rate was determined for all expressed genes. Tukey boxplots show mutations per UTR in percent. Outliers are not shown. Median observed frequency for each individual mutation is indicated. Statistical analysis of increase in T>C conversions was determined by Mann-Whitney test.

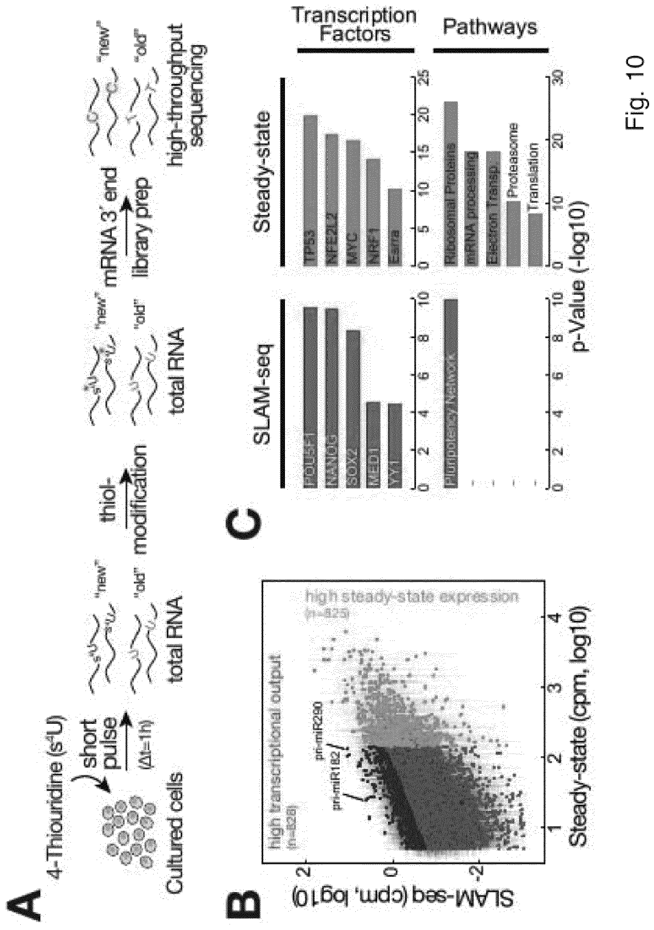

**Figure 10****. Determining the poly-adenylated transcriptional output in mES cells. (A)** Experimental setup to determine the transcriptional output of poly-adenylated mRNA in mES cells by the invention. **(B)** Relative abundance of T>C conversion containing transcripts ("SLAM-seq") and non-T>C conversion containing transcripts ("Steady-state") in counts per million (cpm), detected by mRNA 3' end sequencing as described in (A). Transcripts overrepresented in SLAM-seq relative to steady-state are indicated in red (high transcriptional output; n=828). Most abundant transcripts at steady-state are indicated in yellow (high steady state expression; n=825). Transcripts corresponding to mES cell specific primary-miRNA clusters miR-290 and miR-182 are shown. **(C)** Comparison of 828 genes that were over-represented among newly transcribed RNA (SLAM-seq) to the top 825 genes detected at steady-state by conventional mRNA 3' end sequencing (Steady-state) in terms of predicted underlying transcription factors (using Ingenuity Pathway Analysis; www.Ingenuity.com), as well as molecular pathways (using Enrichr).

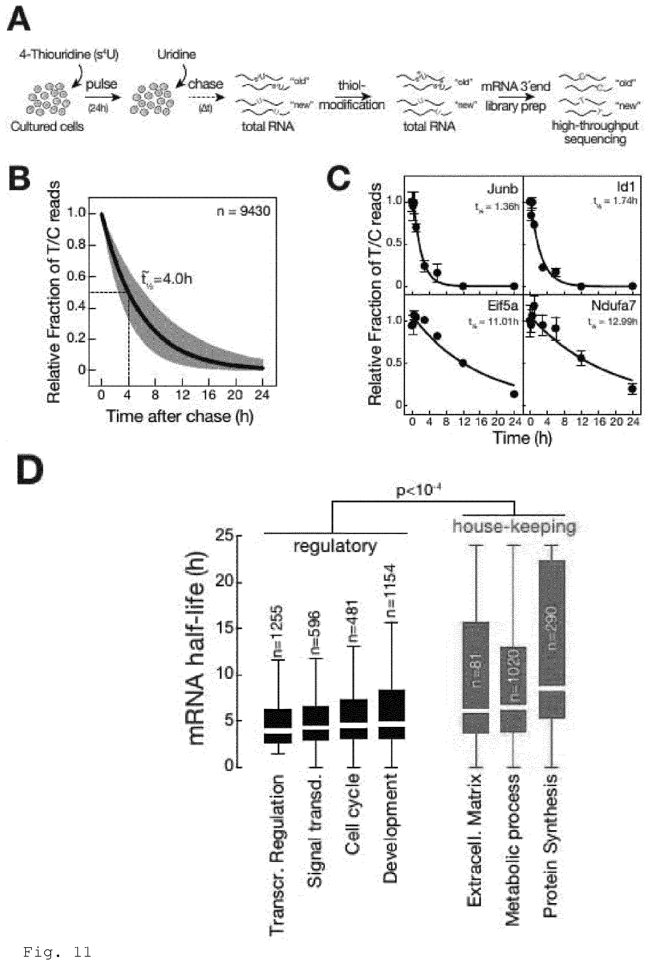

**Figure 11****. Global analysis of mRNA stability in mES cells. (A)** Experimental setup to determine the stability of poly-adenylated mRNA in mES cells by the invention. **(B)** Global analysis of mRNA half-lives in mES cells. The relative fraction of T>C conversion containing reads mapping to annotated 3' UTRs of 9430 abundantly expressed genes in mES cells were normalized to the 24h pulse labeling timepoint and median, upper and lower quartiles over time were fit using single exponential decay kinetics, revealing a median mRNA half-life (^{∼}t_{1/2}) of 4.0 h. **(C)** Half-life calculation for individual example transcripts. The average fraction of T>C conversion containing reads relative to 24h pulse timepoint of three independent replicates for Junb, Id1, Eif5a and Ndufa7 are shown and fit to single exponential decay kinetics. The average half-life (t_{1/2}) for each transcript as determined by curve fitting is indicated. **(D)** Tukey boxplot representation of mRNA half-life determined by mRNA 3' end sequencing for transcripts classified according to their associated GO-terms into regulatory (i.e. transcriptional regulation, signal transduction, cell cycle and development) or house-keeping (i.e. extracellular matrix, metabolic process and protein synthesis). The number of transcripts for each category are indicated. P-value (determined by Mann-Whitney test) is indicated.

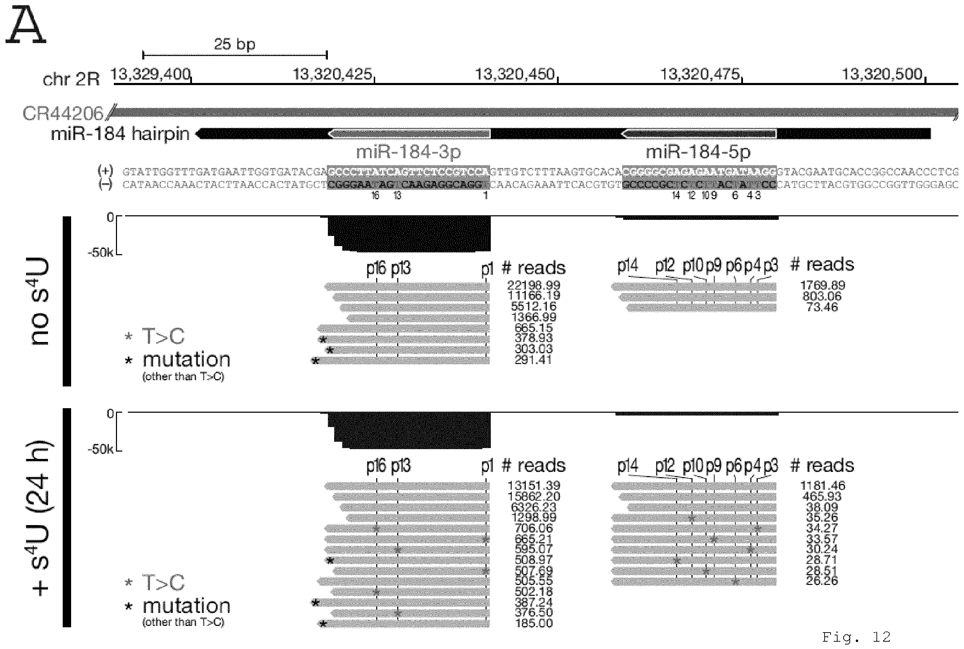

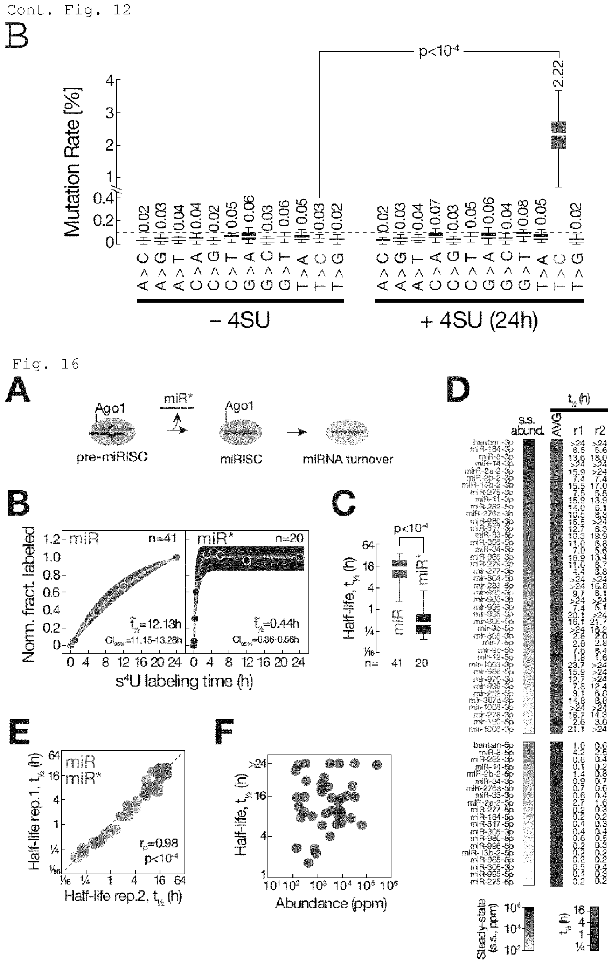

**Figure 12****. Thiol-linked alkylation for the metabolic labeling of small RNAs. (A)** Representative genome browser screen shot for small RNA libraries generated from size-selected total RNA of *Drosophila* S2 cells. A representative area in the *Drosophila melanogaster* genome encoding miR-184 is shown. Nucleotide positions encoding thymine (T, red) are indicated relative to the 5' end of each small RNA species. Reads representing 99% of all 5' isoforms are shown for miR-184-3p and -5p and the respective number of reads are indicated in parts per million (ppm). **(B)** Small RNA sequencing libraries generated from total RNA of *Drosophila* S2 cells before and after s⁴U metabolic labeling for 24 h were mapped to annotated miRNAs and any given mutation rate was determined for abundantly expressed miRNAs (>100 ppm). Tukey boxplots show mutations per miRNA in percent. Outliers are not shown. Median observed frequency for each individual mutation are indicated. P-Value, as determined by Mann-Whitney test is indicated.

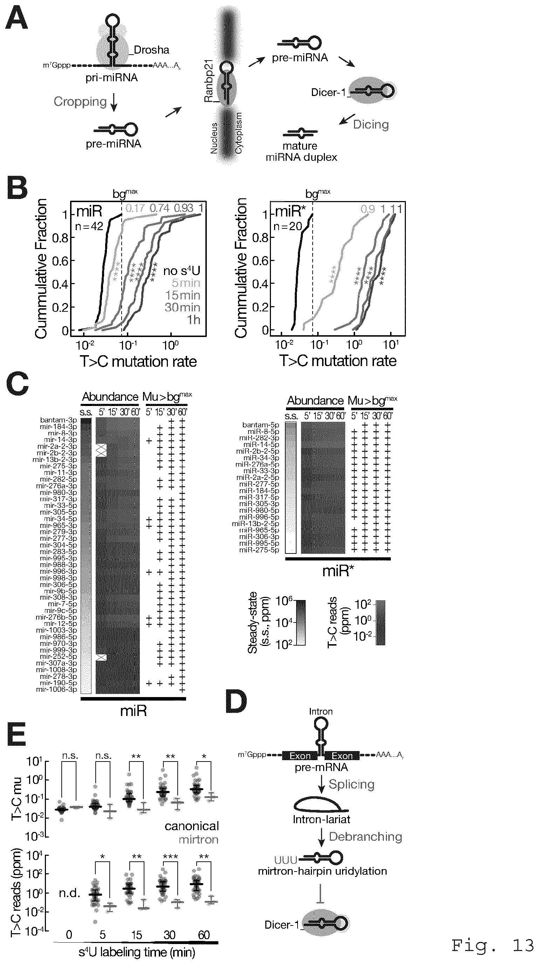

**Figure 13****. Intracellular kinetics of microRNA biogenesis. (A)** MicroRNAs are derived from hairpin-containing RNA polymerase II transcripts (primary microRNAs, pri-miRNAs) through sequential processing by the RNase III enzymes Drosha in the nucleus and Dicer in the cytoplasm resulting in a ~22nt microRNA duplex. The hairpin-processing intermediate (precursor-microRNA, pre-miRNA) is exported from the nucleus to the cytoplasm by Ranbp21 in a RanGTP-dependent manner. **(B)** Cummulative distribution plots show the median T>C mutation rates for 42 abundantly expressed miRNAs (left) or 20 miR*s (right) in small RNA libraries generated from total RNA of *Drosophila ago2*^{ko} S2 cells treated with s⁴U for the indicated time. P-Values were determined by Kolmogorov-Smirnov test (**** = p<10⁻⁴). Bg^{max} indicates the maximum background error rate. **(C)** Average abundance of the indicated miRNAs (left) or miR*s (right) are shown at steady state or T>C conversion containing reads at the indicated timepoints after metabolic labeling by s⁴U. Number of reads normalized to total small RNAs are shown in parts per million (ppm). Excess of mutation rates (Mu) above background maximum (bg^{max}) is indicated. **(D)** Mirtron hairpins are generated through splicing of protein coding transcripts. After intron lariat debranching mirtron hairpins are subjected to post-transcriptional uriylation in the cytoplasm, which modulates the 2nt-3' overhang of pre-mirtrons, preventing miRNA biogenesis by Dicer. **(E)** T>C mutation rates (top) or small RNA-normalized T>C reads in parts per million (ppm) for canonical miRNAs (grey) or mirtrons (red) at the indicated timepoint of a s⁴U-labeling experiment. Median and interquartile range are indicated. P-Value (Mann-Whitney test) is indicated (*, p<0.05; **, p<0.01; ***, p<0.001). N.d., not detected.

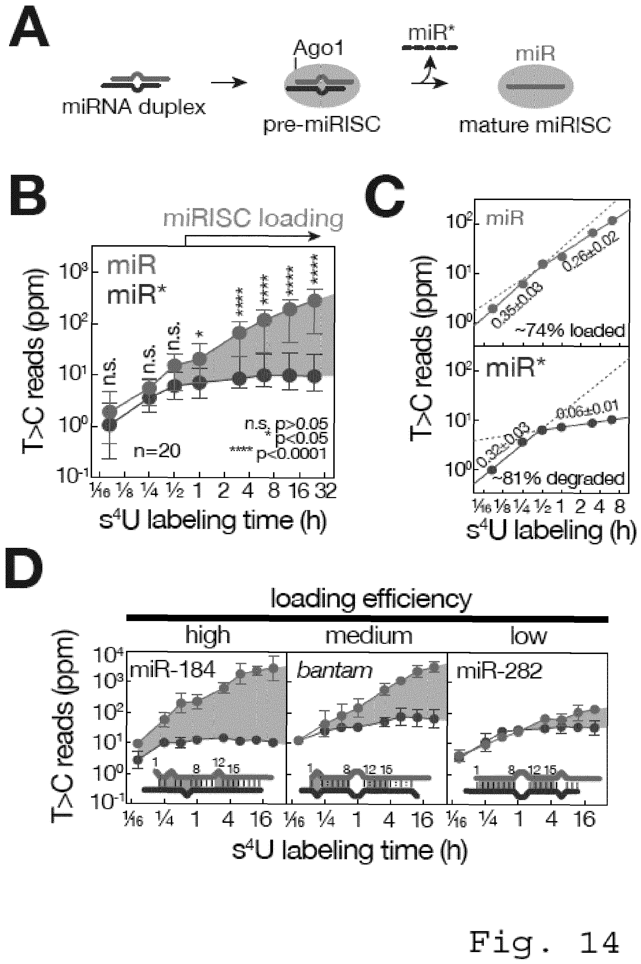

**Figure 14****. Intracellular kinetics of microRNA loading. (A)** Upon production, the microRNA duplex is loaded onto the Argonaute protein Ago1. In this process one of the two strands - the miR strand - is selectively retained in Ago1, whereas the other one - the miR* strand - is expelled and degraded. A single stranded miRNA bound to Ago1 forms the mature miRNA-induced silencing complex (miRISC). **(B)** Median accumulation of T>C conversion containing reads (in ppm) of 20 abundantly expressed miR and miR* pairs in the course of a s⁴U metabolic labeling experiment in *ago2*^{ko} S2 cells. Median and interquartile range for miR (red) and miR* (blue) are shown. Values are derived from two independent measurements. P-value indicates significant separation of miR and miR* as determined by Mann Whitney test (*, p<0.05; ****, p<0.0001). **(C)** Zoom-in for the timecourse shown in (B) for miR (red, top) and miR* (blue, bottom).

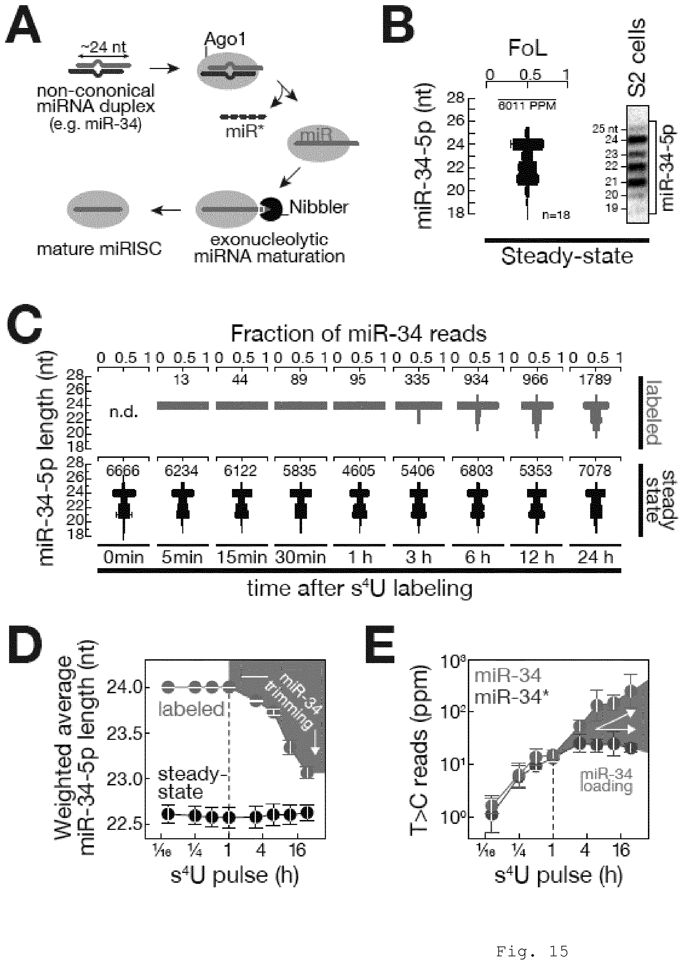

**Figure 15****. Intracellular kinetics of exonucleolytic miRNA trimming. (A)** Model for exonucleolytic miRNA maturation in *Drosophi*la. A set of microRNAs (e.g. miR-34) are produced by Dicer as longer, ∼24 nt miRNA duplexes that, upon loading into Ago1 and removal of the miR* strand, undergo exonucleolytic maturation mediated by the 3'-to-5' exoribonuclease Nibbler to form a mature, gene-regulatory, miRNA induced silencing complex. **(B)** Steady state length distribution of miR-34-5p in *Drosophila ago2*^{ko} S2 cells as determined by high-throughput sequencing of small RNAs (left, bars represent mean ± standard deviation of 18 measurements; the average cloning count is indicated in parts per million, ppm) or Northern-hybridization experiments (right). **(C)** Length distribution of miR-34-5p in libraries prepared from *Drosophila ago2*^{ko} S2 cells subjected to s⁴U metabolic labeling for the indicated time. Length distribution of T>C conversion containing reads (labeled, red, top) and all reads (steady-state, black, bottom) are shown. The underlying number of reads are indicated. Data show mean±standard deviation of two independent replicates. **(D)** Weighted average length of miR-34-5p in libraries prepared from *Drosophila ago2*^{ko} S2 cells subjected to s⁴U metabolic labeling for the indicated time. Data represents mean±standard deviation of T>C conversion-containing reads (labeled, red) and all reads (steady-state, black). Decrease in weighted average length of T>C conversion-containing reads indicates exonucleolytic trimming (highlighted by grey area). **(E)** Loading of miR-34-5p as determined by the relative abundance of T>C conversion containing reads in miR-34-5p (miR-strand, red) and miR-34-3p (miR* strand, blue) after s⁴U metabolic labeling of Drosophila S2 cells. Mean±standard deviation of two independent experiments are shown. Loading is represented by the separation of miR from miR* and highlighted by grey area.
**Figure 16****. Differential stability of miRNAs. (A)** Upon loading of a microRNA duplex onto Ago1, forming pre-miRISC, the miR* strand (blue) is degraded, resulting in a mature miRNA-induced silencing complex (miRISC). The precise stability of miRNAs in miRISCs has remained obscure. **(B)** Increase in per-T-position mutation rate for 41 abundantly expressed miRNAs (red, left) and 20 miR*s (blue, right) across an s⁴U-metabolic labeling timecourse in *Drosophila ago2*^{ko} S2 cells. For each small RNA, the median mutation rate across all T-positions was determined and normalized to the 24-h timepoint. The median and interquartile range is shown. Values represent the mean of two independent replicates. The median half-life (t_{1/2}) and the 95% confidence interval was determined by single-exponential curve fitting. **(C)** Tukey box plot representing the half-life of 41 miRs (red) and 20 miR*s (blue). P-value was determined by Mann-Whitney test. **(D)** Steady-state abundance and average half-life for the indicated miR (red) and miR* (blue). Average half-life represents the mean of two independent replicates. The individual half-life measurements for two independent experiments (r1 and r2) are reported. Half-life data that exceeded the total time of the measurement are indicated as >24h. **(E)** Comparison of half-life values determined in two independent biological replicates for 41 miR (red) and 20 miR* (blue) strands. Pearson's correlation coefficient (r_{P}) and associated p-value are shown. **(F)** MicroRNA stability differentially contributes to steady-state abundance of miRNAs. Half-life values for 40 miR are shown relative to their steady-state abundance. Data represents mean of two independent biological replicates.

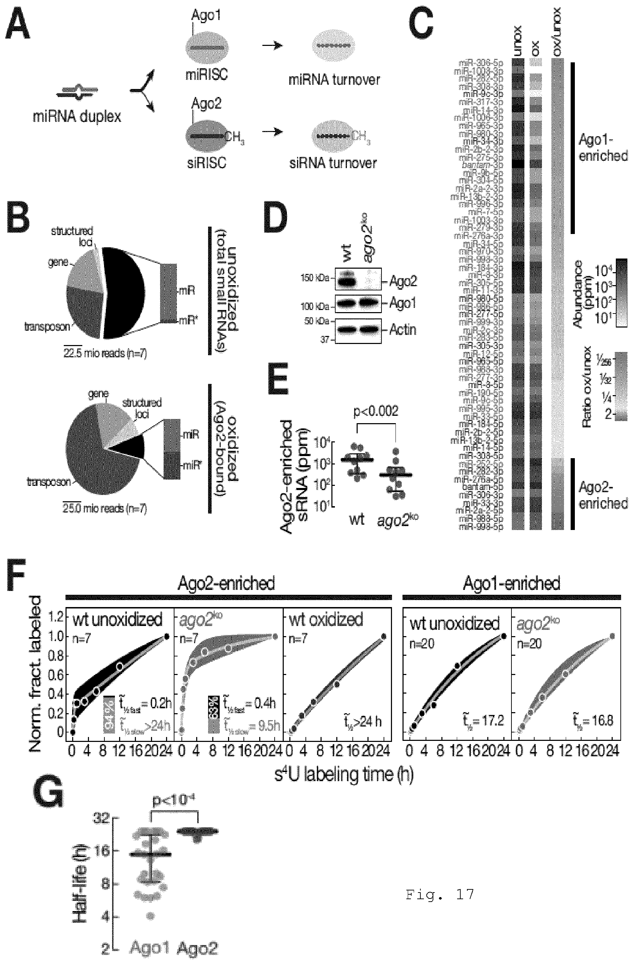

**Figure 17****. Argonaute protein identity determines small RNA stability. (A)** In *Drosophila,* miRNAs preferentially load onto Ago1 to form miRISC. In parallel, a subset of miR*s load into Ago2 to form siRISC. siRISC formation is accompanied by the specific methylation of Ago2-bound small RNAs at the 2' position of the 3' terminal ribose. If Argonaute protein identity differentially affects small RNA stability is unknown. **(B)** Pie charts represent the relative abundance of different endo-siRNA classes and miRNAs in small RNA libraries from wild-type *Drosophila* S2 cells. Results from a standard cloning protocol (unoxidized, upper diagram) and from a cloning strategy that enriches for small RNAs with modified 3' termini (oxidized, lower diagram) are shown. The fraction of miRs and miR*s is indicated for both libraries. The average distribution of 7 datasets is shown. The average library depth is indicated. **(C)** Heat maps show the relative abundance of miRs (red), and miR*s (blue) in the indicated libraries (in grayscale). The ratio of relative representations in the libraries indicates preferential association of small RNAs with either AGO1 (green) or AGO2 (red). **(D)** Western blot analysis of wild-type (wt) S2 cells, or S2 cells depleted of Ago2 by CRISPR/Cas9 genome engineering (*ago2*^{ko}). Actin represents a loading control. **(E)** Relative abundance of Ago2-enriched miR and miR* in wild-type (wt) and *ago2*^{ko} Drosophila S2 cells. Median and interquartile range is indicated. P-value was determined by Wilcoxon matched-pairs signed rank test. **(F)** Decay kinetics of Ago2- (left) and Ago1-enriched small RNAs (right) in standard libraries prepared from an s⁴U metabolic labeling timecourse in wild-type (wt, black) or *ago2*^{ko} S2 cells (red) or from wild-type S2 cells employing a cloning strategy that enriches for small RNAs with modified 3' termini (wt oxidized, blue). Median and interquartile range of two-phase or one-phase exponential fit (as specified in main text) are shown. The half-life (t1/2) as determined by curve-fitting is indicated. In case of two-phase kinetics, the relative contribution of fast and slow kinetics is shown. **(G)** Half-life of the 30 most abundant miRNAs in ago2ko S2 cells (red, Ago1) or the most abundant miRs and miR*s in small RNA libraries employing a cloning strategy that enriches for small RNAs with modified 3' termini (blue, Ago2). The median and interquartile range is indicated. P-value was determined by Mann-Whitney test.

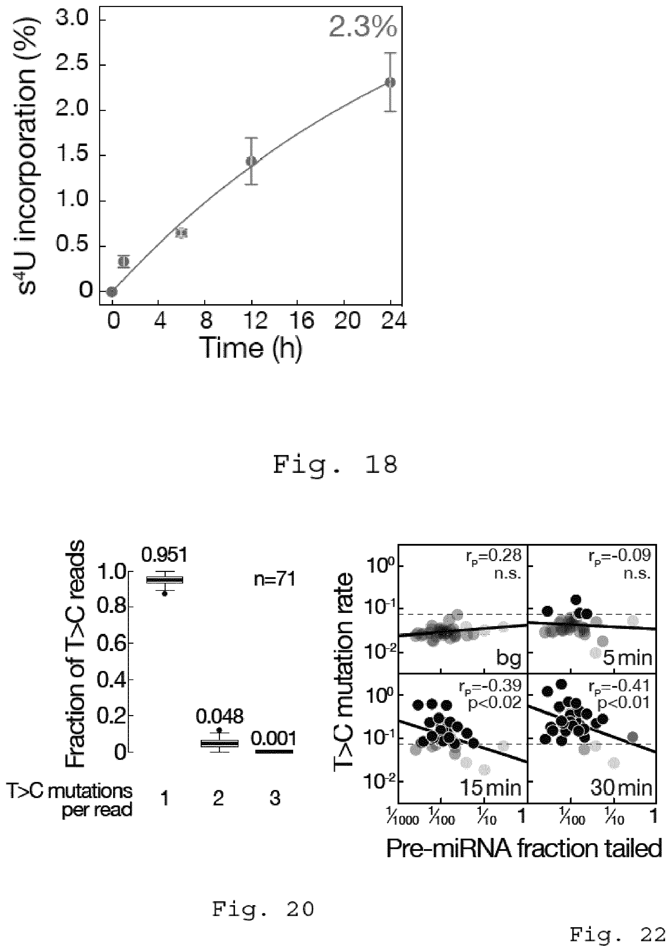

**Figure 18****. 4-thiouridine metabolic labeling in *Drosophila* S2 cells.** Quantification of s⁴U-incorporation into total RNA after s⁴U-metabolic labeling for the indicated time in a pulse labeling experiment in *Drosophila* S2 cells. Substitution rate of s⁴U compared to unmodified uridine determined by HPLC is shown and was determined as previously described (Spitzer et al. (2014) Meth Enzymol 539, 113-161.). Values represent mean±SD of three independent replicates. Maximum incorporation rates after 24 h labeling are indicated.

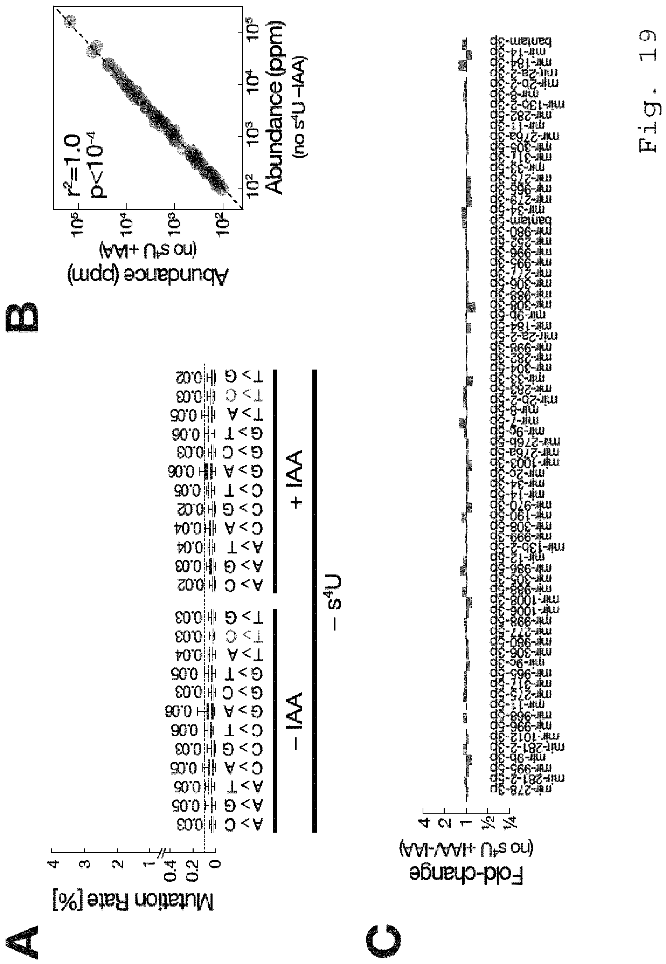

**Figure 19****. Iodoacetamide treatment does not affect the quality of small RNA libraries.** Small RNA sequencing libraries generated from total RNA of *Drosophila* S2 cells before and after treatment with iodoacetamide were mapped to annotated miRNAs and abundantly expressed miRNAs (>100 ppm) were analyzed. **(A)** Any given mutation rate was determined for each miRNA from small RNA libraries of iodoacetamide-treated or untreated total RNA. Tukey boxplots show mutations per miRNA in percent. Outliers are not shown. Median observed frequency for each individual mutation are indicated. **(B)** Abundance of miRNAs in small RNA libraries prepared from iodoacetamide-treated or untreated total RNA. Pearson correlation coefficient and associated p-value is indicated. **(C)** Fold-change in expression for individual miRNAs in small RNA libraries prepared from iodoacetamide-treated or untreated total RNA.
**Figure 20****. Frequency of s⁴U-incorporation in metabolically labeled small RNAs.** Tukey boxplots show the fraction of T>C conversion reads containing one, two, or three T>C mutations for each of 71 abundantly expressed (>100 ppm) miRNAs in small RNA libraries prepared from size selected total RNA of Drosophila S2 cells subjected to s⁴U metabolic labeling for 24 h. The median fraction of T>C conversion reads is indicated.

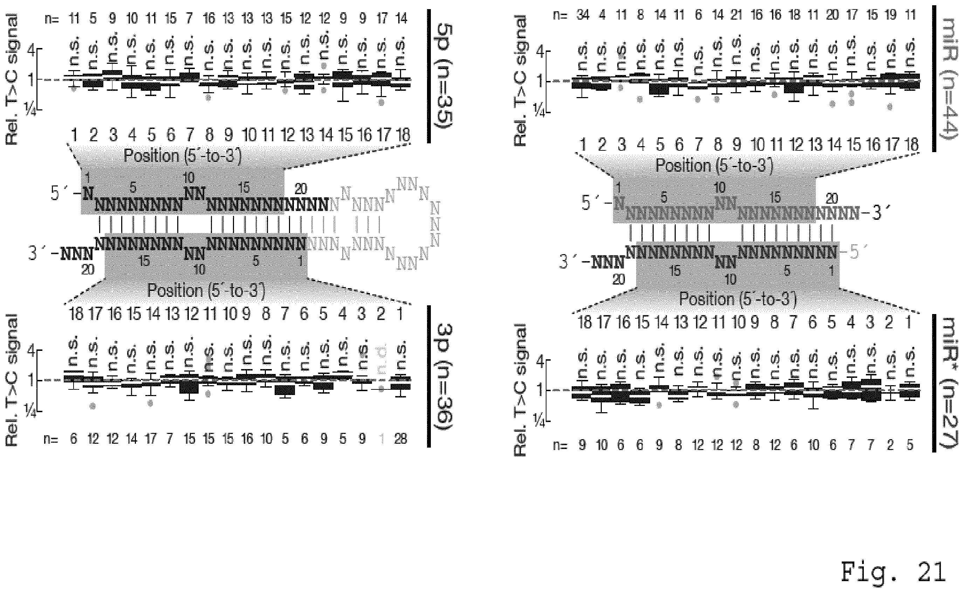

**Figure 21****. s⁴U-metabolic labeling does not impact microRNA biogenesis or loading.** Over- or underrepresentation of T>C conversions at individual positions of a given small RNA that is derived from the 5p- or 3p arm of a microRNA precursor (left), or that constitutes a miR or miR* strand, as defined by selective Argonaute-loading (right). Results are derived from 71 abundantly expressed (>100 ppm) microRNAs (corresponding to 35 5p- and 36 3p-miRNAs, or 44 miR and 27 miR*). Statistically significant differences in relative representation were compared to the total population for the indicated position by Mann-Whitney test. n.s., p>0.05; n.d., not determined due to limited data points.
**Figure 22****. Precursor-miRNA tailing counteracts efficient miRNA biogenesis.** Correlation between pre-miRNA uridylation and T>C mutation rates in SLAM-seq small RNA libraries prepared from *ago2*^{ko} S2 cells after treatment with s⁴U for the indicated time. Pearson's correlation coefficient (r_{P}) and associated p-Value are shown.

### Examples:

### Example 1: Materials and Methods

### Carboxyamidomethylation of s⁴U

If not indicated otherwise, carboxyamidomethylation was performed under standard conditions (50% DMSO, 10 mM iodoacetamide, 50 mM sodiumphosphate buffer pH8, for 15min at 50°C) using either 1 mM 4-thiouracil (SIGMA), 800 µM 4-thiouridine (SIGMA), or 5 - 50 µg total RNA prepared from s⁴U metabolic labeling experiments. The reaction was quenched by addition of excess DTT.

### Adsorption measurements

1mM 4-thiouracil was incubated under optimal reaction conditions (10mM iodoacetamide, 50% DMSO, 50 mM sodiumphosphate buffer pH8, for 15min at 50°C) if not indicated otherwise. Reaction was quenched by the addition of 100 mM DTT and adsorption spectra were measured on a Nanodrop 2000 instrument (Thermo Fisher Scientific), followed by baseline subtraction of adsorption at 400 nm.

### Mass Spectrometry

40 nmol 4-thiouridine were reacted in the absence or presence of 0.05, 0.25, 0.5 or 5 µmol iodoacetamide under standard reaction conditions (50 mM sodiumphosphate buffer, pH 8; 50 % DMSO) at 50°C for 15min. The reaction was stopped with 1% acetic acid. Acidified samples were separated on a Ulitimate U300 BioRSLC HPLC system (Dionex; Thermo Fisher Scientific), employing a Kinetex F5 Pentafluorophenyl column (150 mm x 2.1 mm; 2.6 µm, 100 Å; Phenomenex) with a flow rate of 100 µl/min. Nucleosides were on-line analyzed using a TSQ Quantiva mass spectrometer (Thermo Fisher Scientific) after electrospray ionization with the following SRMs: 4-Thiouridine m/z 260 → 129, alkylated 4-Thiouridine m/z 318 → 186, 6-Thio-Guanosinem/z 300 → 168 and alkylated 6-Thio-Guanosine m/z 357 → 225. Data were interpreted using the Trace Finder software suite (Thermo Fisher Scientific) and manually validated.

### Primer extension assays

Primer extension assays were essentially performed as described previously by Nilsen et al. (Cold Spring Harb Protoc. 2013, 1182-1185). Briefly, template RNA oligonucleotides (5L-let-7-3L or 5L-*let*-7-s⁴Up9-3L; Dharmacon; see Table for sequences) were deprotected according to the instructions of the manufacturer and purified by denaturing polyacrylamide gel-elution. 100 µM purified RNA oligonucleotides were treated with 10 mM iodoacetamide (+IAA) or EtOH (-IAA) in standard reaction conditions (50 % DMSO, 50 mM sodiumphosphate buffer, pH 8) for 15min at 50°C. The reaction was stopped by addition of 20 mM DTT, followed by ethanol precipitation. RT primer (see Table for sequence) was 5' radiolabeled using γ-³²P-ATP (Perkin-Elmer) and T4-polynucleotide kinase (NEB), followed by denaturing polyacrylamide gel-purification. 640 nM γ-³²P-RT primer was annealed to 400 nM 5L-let-7-3L or 5L-*let*-7-s⁴Up9-3L in 2 x annealing buffer (500 mM KCl, 50 mM Tris pH 8.3) in a PCR machine (3min 95°C, 30 sec 85°C Ramp 0.5°C/s, 5min 25°C Ramp 0.1°C/s). Reverse transcription was performed using Superscript II (Invitrogen), Superscript III (Invitrogen), or Quant-seq RT (Lexogen) as recommended by the manufacturer. For dideoxynucleotide reactions, a final concentration of 500 µM ddNTP (as indicated) was added to RT reactions. Upon completion, RT reactions were resuspended in formamide loading buffer (Gel loading buffer II, Thermo Fisher Scientific) and subjected to 12.5% denaturing polyacrylamide gel electrophoresis. Gels were dried, exposed to storage phosphor screen (PerkinElmer), imaged on a Typhoon TRIO variable mode imager (Amersham Biosciences), and quantified using ImageQuant TL v7.0 (GE Healthcare). For analysis of drop-off, signal-intensities at p9 were normalized to preceding drop-off signal intensities (bg, Figure 4B) for individual reactions. Values reporting the change in drop off signal (+IAA/-IAA) for s⁴U-containing and non-containing RNA oligonucleotides were compared for the indicated reverse transcriptases.

**Table. RNA oligonucleotides used for primer extension assay. s⁴U indicates 4-thiouridine. Inspected sequence of let-7 is indicated in red.**

| **Name** | **RNA sequence (5'-3')** |
|---|---|
| 5L-let-7-3L | ACACUCUUUCCCUACACGACGCUCUUCCGAUCUUGAGGUAGUA GGUUGUAUAGUAGAUCGGAAGAGCACACGUCUC (SEQ ID NO: 1) |
| 5L-let-7-s⁴U-p9-3L | ACACUCUUUCCCUACACGACGCUCUUCCGAUCUUGAGGUAGs⁴ UAGGUUGUAUAGUAGAUCGGAAGAGCACACGUCUC (SEQ ID NO: 2) |
| | |

| DNA oligonucleotide used for primer extension assay. | |
|---|---|
| **Name** | **DNA sequence (5'-3')** |
| RT primer | GTGACTGGAGTTCAGACGTGTGCTCTTCCGATCT (SEQ ID NO: 3) |
| | |

| DNA oligonucleotides used for cDNA amplification followed by Illumina high-throughput sequencing. Barcode nucleotides arein- dicated in red. | |
|---|---|
| **Name** | **DNA sequence (5'-3')** |
| Solexa_PCR_fwd | AATGATACGGCGACCACCGAGATCTACACTCTTTCCCTACACG ACGCTCTTCCGATCT (SEQ ID NO: 4) |
| Solexa_IDX_rev | CAAGCAGAAGACGGCATACGAGATNNNNNNGTGACTGGAGTTC AGACGTGTGCTCTTCCGATCT (SEQ ID NO: 5) |

### HPLC analysis of s⁴U-labeled RNA

Analysis of s⁴U-incorporation into total RNA following metabolic labeling was performed as previously described by Spitzer et al. (Meth Enzymol. 539, 113-161 (2014)).

### Cell viability assay

5000 mES cells were seeded per 96 well the day before the experiment. Medium containing different concentrations of s⁴U (as indicated) was added to the cells for 12h or 24h. Cell viability was assessed by CellTiter-Glo® Luminescent Cell Viability Assay (Promega) according to the instructions of the manufacturer. Luminescent signal was measured on Synergy using Gen5 Software (v2.09.1).

### Cell culture

Mouse embryonic stem (mES) cells (clone AN3-12) were obtained from Haplobank (Elling et al., WO2013/079670) and cultured in 15 % FBS (Gibco), 1x Penicillin-Streptomycin solution (100 U/ml Penicillin, 0.1 mg/ml Streptomycin, SIGMA), 2 mM L-Glutamine (SIGMA), 1x MEM Non-essential amino acid solution (SIGMA), 1 mM sodium pyruvate (SIGMA), 50 µM 2-Mercaptoethanol (Gibco) and 20 ng/ml LIF (in-house produced). Cells were maintained at 37° C with 5% CO₂ and passaged every second day.

### Modification of RNA to alter sequencing ("SLAM-seq")

mES cells were seeded the day before the experiment at a density of 10⁵ cells/ml in 10 cm dishes. s⁴U-metabolic labeling was performed by incubation of mES cells in standard medium but adding 100 µM s⁴U (SIGMA) from a 500 mM stock solution in water. During the metabolic labeling, s⁴U containing medium was exchanged every 3h. For the uridine chase experiment, s⁴U containing medium was discarded, cells were washed twice with 1x PBS and incubated with standard medium supplemented with 10 mM uridine (SIGMA). Cells were directly lysed in TRIzol® (Ambion) and RNA was extracted following the manufacturer instructions except that 0.1 mM final concentration of DTT was added during isopropanol precipitation. RNA was resuspended in 1 mM DTT. 5 µg of total RNA were treated with 10 mM iodoacetamide under optimal reaction conditions and subsequently ethanol precipitated and subjected to QuantSeq 3' end mRNA library preparation (Moll et al., Nat Methods 11 (2014); WO2015/140307).

### RNA library preparation

Standard RNA seq libraries were prepared using NEBNext® Ultra™ Directional RNA Library Prep Kit for Illumina® (NEB) following the instructions of the manufacturer. Cap-seq libraries were prepared as previously described by Mohn et al. (Cell. 157, 1364-1379 (2014)) except that ribosomal RNA depletion using magnetic RiboZero Kit (Epicenter) was performed prior to fragmentation. Messenger RNA 3' end sequencing was performed using the Quant-seq mRNA 3' end library preparation kit (Lexogen) according to the instructions of the manufacturer.

### Data analysis

Gel images were quantified using ImageQuant v7.0a (GE Healthcare). Curve fitting was performed according to the integrated rate law for a first-order reaction in Prism v7.0 (GraphPad) or R (v2.15.3). Statistical analyses were performed in Prism v7.0a (GraphPad), Excel v15.22 (Microsoft) or R (v2.15.3).

### Bioinformatics

For sequencing analysis of synthetic RNA samples (Figure 5) barcoded libraries were demultiplexed using Picard Tools BamIndexDecoder v1.13 allowing 0 mismatches in the barcode. Resulting files were converted to fastq using picard-tools SamToFastq v1.82. Cutadapt v1.7.1 was used to trim adapters (allowing for default 10% mismatch in adapter sequence) and filter for sequences of 21nt length. Resulting sequences were aligned to mature dme-let-7 sequence (5'-TGAGGTAGTAGGTTGTATAGT-3', SEQ ID NO: 6) using bowtie v0.12.9 allowing for 3 mismatches and converted to bam using samtools v0.1.18. Sequences containing ambiguous nucleotides (N) were removed. Remaining aligned reads were converted to pileup format. Finally, the fraction of each mutation per position was extracted from pileup. Output table was analyzed and plotted in Excel v15.22 (Microsoft) and Prism v7.0a (GraphPad).

For mRNA 3' end sequencing data analysis, barcoded libraries were demultiplexed using Picard Tools BamIndexDecoder v1.13 allowing 1 mismatch in the barcode. Adapters were clipped using cutadapt v1.5 and reads were size-filter for ≥ 15 nucleotides. Reads were aligned to mouse genome mm10 using STAR aligner v2.5.2b. Alignments were filtered for alignment scores ≥0.3 and alignment identity ≥ 0.3 normalized to read length. Only alignments with ≥30 matches were reported. Only chimeric alignments with an overlap ≥15 bp were allowed. 2-pass mapping was used. Introns <200 kb were filtered, alignments containing non-canonical junctions were filtered. Alignment with a mismatch to mapped bases ratio ≥ 0.1 or with a maximum number of 10 mismatches were filtered. The maximum number of gaps allowed for junctions by 1,2,3,N reads was set to 10 kb, 20kb, 30kb and 50 kb, respectively. The minimum overhang length for splice junctions on both sides for (1) non-canonical motifs, (2) GT/AG and CT/AC motif, (3) GC/AG and CT/GC motif, (4) AT/AC and GT/AT motif was set to 20, 12, 12, 12, respectively. "Spurious" junction filtering was used and the maximum number of multiple alignments allowed for a read was set to 1. Exonic reads (Gencode) were quantified using FeatureCounts.

For Cap analysis gene expression (CAGE), barcoded libraries were demultiplexed using Picard Tools BamIndexDecoder v1.13 allowing 1 mismatch in the barcode. The first 4 nt of the reads were trimmed using seqtk. Reads were screened for ribosomal RNA by aligning with BWA (v0.6.1) against known rRNA sequences (RefSeq). The rRNA subtracted reads were aligned with TopHat (v1.4.1) against the Mus musculus genome (mm10). Maximum multihits were set to 1, segment-length to 18 and segment-mismatch to 1. Additionally, a gene model was provided as GTF (Gencode VM4).

For analysis of mRNA 3' end sequencing (Quant-seq) datasets, reads were demultiplexed using Picard Tools BamIndexDecoder v1.13 allowing 1 mismatch in the barcode. Five nucleotides at the 5' end of demultiplexed reads were trimmed. Reads were aligned to mouse genome mm10 and alignments in annotated 3' UTRs (Gencode) were counted using SLAMdunk (Neumann & Rescheneder, manuscript in preparation). Briefly, SLAMdunk relies on NextGenMap, a flexible and fast read mapping program, and was tailored using an adapted scoring scheme eliminating T>C mismatch penalties for the mapping step. T>C containing reads and non-T>C containing reads aligning to 3' UTRs were quantified to deduce s⁴U- or unlabeled transcript abundance, respectively. For transcriptional output analysis, the number of normalized reads (in cpm; "Steady-state Expression") and the number of normalized reads containing ≥1 T/C mutation (in cpm; "Transcriptional Output") were obtained for every gene after aligning the high-throughput sequencing data with SLAMdunk to the mouse genome mm10. Mitochondrial (Mt-) and predicted (GM-) genes were excluded from the analysis. Background T/C reads (T/C reads observed without s⁴U labeling) were subtracted from the T/C reads in the 1h time-point and an expression threshold of >5 cpm for the mean of "Steady-state Expression" was set. To identify genes with a high transcriptional output, a linear regression was fitted after plotting log10 (SteadyState Expression) vs. log10 (Transcriptional Output) (number of genes: 6766), described by the equation Y = 0.6378*X - 1.676. For each gene, the distance to the fitted curve was calculated ("ΔY") as in ΔY = TranscriptionalOutput(cpm) - (0.6378 * SteadystateExpression (cpm) -1.676). "High transcriptional output" genes were defined by ΔY > 0.5 (number of genes: 828). "High expression genes" were defined by steady-state CPM > log10(2.15) (number of genes: 825). To predict the transcription factor network defining each class of genes, Ingenuity Pathway Analysis (Qiagen) v27821452, a web-delivered application that enables biologists to discover, visualize and explore therapeutically relevant networks significant to their experimental results, such as gene expression data sets, was used with the input of "High transcriptional Output" or "High expression" genes. For a detailed description of Ingenuity Pathways Analysis visit www.Ingenuity.com. The top 5 predicted upstream regulators are shown.

To predict pathways of "High transcriptional Output" or "High expression" genes, the online tool Enrichr was used with the input of the two gene classes. The top 5 predicted pathways are displayed.

### Example 2: Thiol-linked alkylation for the metabolic sequencing of RNA

As proof-of-principle thiol nucleotide-analog was selected as example of a derivatization strategy that bypasses the need for biochemical separation of s⁴U-labeled and unlabeled RNA species to determine RNA expression kinetics in cultured cells (Figure 1): This strategy is based on well-established metabolic labeling approaches but avoids the ineffective and time-consuming biotinylation-step. It contains a short chemical treatment protocol that involves the modification of s⁴U-containing RNA with iodoacetamide, a sulfhydryl-reactive compound, which - upon reaction with s⁴U - creates a bulky group at the base-pairing interface (Figure 1). When combined with well-established RNA library preparation protocols, the presence of the bulky group at the sites of s⁴U -incorporation leads to the specific and quantitative misincorporation of G during reverse transcription (RT), but does not interfere with RT-processivity. s⁴U-containing sequences can therefore be identified by sequence comparison or bioinformatically in high-throughput sequencing libraries at single nucleotide resolution by calling T to C transitions. Importantly, no enzyme that converts uridine to cytosine is known and similar error rates (i.e. T>C conversions) in high throughput RNA sequencing datasets using e.g. the Illumina HiSeq 2500 platform are rare, occurring at a frequency of less than one in a ten thousand. This approach is referred to "SLAM-seq" as abbreviation of its most preferred embodiment, the thiol(SH)-linked alkylation for the metabolic sequencing of RNA.

SLAM-seq is based on nucleotide-analog derivatization chemistry that enables to detect metabolic-labeling-derived 4-thiouridine-incorporation events in RNA species at single-nucleotide resolution by high-throughput sequencing. We show that the new method accurately measures RNA polymerase II-dependent poly-adenylated transcriptional output, and recapitulates global post-transcriptional gene regulatory signatures in mouse embryonic stem cells. The invention provides a scalable, highly quantitative, cost- and time-effective method for the rapid and transcriptome-wide analysis of RNA expression kinetics at high temporal resolution.

For s⁴U-derivatization, we employed iodoacetamide (IAA) as an example of an effective primary thiol-reactive compound, attaching a carboxyamidomethyl-group to the thiol group as a result of a nucleophilic substitution (S_{N}2) reaction (Figure 2A). To quantitatively monitor the efficiency of s⁴U-derivatization as a function of different parameters (i.e. time, temperature, pH, IAA-concentration, and DMSO) we monitored the characteristic absorption spectrum of 4-thiouracil (∼335 nm), which - upon reaction with iodoacetamide (IAA) - shifts to ∼297 nm (Figure 2B to K) (45). Under optimal reaction conditions (10 mM IAA; 50 mM NaPO₄, pH8; 50% DMSO; 50°C; 15min) absorption at 335 nm decreases 50-fold compared to untreated 4-thiouracil, resulting in an alkylation rate of at least 98% (Figure 2L and M). (Note, that conversion rates may be underestimated since the absorption spectrum of 4-thiouracil and its alkylated derivative partially overlap.) Analysis of thiol-specific alkylation in a ribose context (i.e. 4-thiouridine) by mass-spectrometry confirmed a close to complete derivatization efficiency (Figure 3).

The quantitative recovery of s⁴U incorporation events presumes that reverse transcriptases pass alkylated s⁴U-residues without drop-off. To determine the effect of s⁴U-alkylation on reverse transcriptase-processivity we employed a synthetic RNA (for sequence see Example 1, Table) that contains a single s⁴U incorporation and assayed three commercially available reverse transcriptases (RTs) - Superscript II, Superscript III, and Quant-seq RT - in primer extension assays (Figure 4A). When normalized to background drop-off signal, we did not observe a significant effect of s⁴U-alkylation on RT processivity when compared to a non-s⁴U-containing oligo with identical sequence (Figure 4B and C). We concluded that s⁴U-alkylation does not result in pre-mature termination of reverse transcription.

In order to evaluate the effect of s⁴U-alkylation on reverse transcriptase-directed nucleotide incorporation, we isolated the full-length products of primer extension reactions, PCR amplified the cDNA and subjected the libraries to high-throughput sequencing using an Illumina HiSeq2500 instrument (Figure 5A). As expected, uridine was accurately reverse transcribed by all three RTs in the non-s⁴U-containing control RNA, irrespective of its treatment with iodoacetamide with average mutation rates of less than 10⁻² (Figure 5B, left panels). In contrast, the presence of s⁴U prompted a constant 10% to 11% T to C conversion even in the absence of alkylation, presumably due to base-pairing variations of s⁴U-tautomeres (Figure 5B, right top panels). Notably, alkylation of s⁴U by iodoacetamide-treatment prompted an 8.5-fold increase in T to C conversion, resulting in a mutation rate of more than 0.94 across all tested RTs (Figure 5B right bottom panels). When compared to reported sequencing errors in Illumina high-throughput sequencing datasets (below 10⁻³) we obtained a signal-to-noise ratio of >940:1. Importantly, we did not observe a significant effect of iodoacetamide-treatment on mutation rates of any given non-thiol-containing nucleotide (Figure 5C). We concluded that iodoacetamide treatment followed by reverse transcription enables the quantitative identification of s⁴U-incorporations in RNA at single nucleotide resolution while not affecting the sequence information of non-thiol-containing nucleotides.

### Example 3: s⁴U-incorporations in metabolically labeled mRNA in mES cells

We tested the ability of mouse embryonic stem cells to tolerate s⁴U metabolic RNA-labeling after 12h or 24h at varying s⁴U concentrations (Figure 6A). As reported previously, high concentrations of s⁴U compromised cell viability with an EC₅₀ of 3.1mM or 380 µM after 12h or 24h labeling, respectively (Figure 6A). Hence, we employed labeling conditions of 100µM s⁴U, which did not severely affect cell viability. Under these conditions, we detected a steady increase in s⁴U-incorporation in total RNA preparation 3h, 6h, 12h, and 24h post labeling, as well as a steady decrease 3h, 6h, 12h, and 24h after uridine chase (Figure 6B). As expected, the incorporation follows a single exponential kinetics, with a maximum average incorporation of 1.78% s⁴U, corresponding to one s⁴U incorporation in every 56 uridines in total RNA (Figure 6C). These experiments establish s⁴U-labeling conditions in mES cells, which can be employed to measure RNA biogenesis and turnover rates under unperturbed conditions.

To test the ability of the method to uncover s⁴U incorporation events in high throughput sequencing datasets we generated mRNA 3' end libraries (employing Lexogen's QuantSeq, 3' mRNA-sequencing library preparation kit) using total RNA prepared from cultured cells following s⁴U-metabolic RNA labeling for 24h (Figure 7) (Moll et al., supra). Quant-seq 3' mRNA-Seq Library Prep Kit generates Illumina-compatible libraries of the sequences close to the 3'end of the polyadenylated RNA, as exemplified for the gene Trim28 (Figure 8A). In contrast to other mRNA-sequencing protocols, only one fragment per transcript is generated and therefore no normalization of reads to gene length is needed. This results in accurate gene expression values with high strand-specificity.

Furthermore, sequencing-ready libraries can be generated within only 4.5h, with ∼2h hands-on time. When combined with the invention, Quant-seq facilitates the accurate determination of mutation rates across transcript-specific regions because libraries exhibit a low degree of sequence-heterogeneity. Indeed, upon generating libraries of U-modified RNA through the Quant-seq protocol from total RNA of mES cells 24h after s⁴U metabolic labeling we observed a strong accumulation of T>C conversions when compared to libraries prepared from total RNA of unlabeled mES cells (Figure 8B). In order to confirm this observation transcriptome-wide, we aligned reads to annotated 3' UTRs and inspected the occurrence of any given mutation per UTR (Figure 9). In the absence of s⁴U metabolic labeling, we observed a median mutation rate of 0.1% or less for any given mutation, a rate that is consistent with Illumina-reported sequencing error rates. After 24h of s⁴U metabolic labeling, we observed a statistically significant (p<10⁻⁴, Mann-Whitney test), 25-fold increase in T>C mutation rates, while all other mutations rates remained below expected sequencing error rates (Figure 9). More specifically, we measured a median s⁴U-incorporation of 2.56% after 24h labeling, corresponding to one s⁴U incorporation in every 39 uridines. (Note, that median incorporation frequency for mRNA are higher than estimated by HPLC in total RNA [Figure 6C], most certainly because stable non-coding RNA species, such as rRNA, are strongly overrepresented in total RNA.) These analyses confirm that the new method uncovers s⁴U-incorporation events in mRNA following s⁴U-metabolic RNA labeling in cultured cells.

### Example 4: Determining the poly-adenylated transcriptional output in mES cells

To test if short s⁴U pulse labeling followed by mRNA 3' end sequencing accurately reports the poly-adenylated transcriptional output, we subjected mES cells to a short, 1h s⁴U-pulse followed by total RNA extraction and mRNA 3' end library preparation (Figure 10A). Quant-seq-generated libraries were mapped to annotated 3' UTRs in the mouse genome and analyzed for the presence of T>C conversions, representing newly transcribed RNA (Figure 10A). When comparing the relative abundance of newly transcribed (i.e. T>C conversion containing) to steady-state (i.e. T>C and non-T>C conversion containing) poly-adenylated transcripts, we observed a subset of transcripts consisting of 828 genes that were over-represented among newly transcribed RNA (Figure 10B). Among the top over-represented transcripts were mES cell-specific microRNA clusters (miR-290- and miR182 cluster), which are thought to be particularly short-lived because they undergo rapid decay upon excision of microRNA hairpins by Drosha in the nucleus, hence do not accumulate to high levels at steady-state (Figure 10B). To more systematically characterize the de novo transcriptional output measured by the new method, we performed gene list enrichment analysis of the 828 genes that were over-represented among newly transcribed RNA as well as the top 825 genes detected at steady-state by conventional mRNA 3' end sequencing in terms of predicted underlying transcription factors (using Ingenuity Pathway Analysis, www.Ingenuity.com), as well as associated molecular pathways (using Enrichr) (Figure 10C): As expected, high-level steady-state expression failed to predict the pluripotency-associated transcription factor network and mostly associated with house-keeping pathways, such as ribosomal proteins, mRNA processing or electron transport. In contrast, de novo transcript analysis by the inventive method successfully predicted key mES cell-specific transcription factors, including Oct4 (POU5F1), NANOG and SOX2, as well as the pluripotency network (Figure 10C). We concluded that short s⁴U-pulse labeling in combination with RNA modification and mRNA 3' end sequencing enables to uncouple the immediate transcriptional output from transcript stability effects, hence provides a rapid and scalable method to study transcriptional gene regulation.

### Example 5: Measuring mRNA transcript stabilities

To determine if the inventive method can be employed to measure mRNA transcript stabilities we performed s⁴U labeling of RNA in mES cells for 24h, followed by a chase using an excess of non-thiol containing uridine, and prepared total RNA at various timepoints (0min, 15min, 30min, 1h, 3h, 6h, 12h, and 24h) followed by U-modification and mRNA 3' end sequencing (Figure 11A). Again we mapped libraries to annotated 3' UTRs in the mouse genome and analyzed them for the presence of T>C conversions, representing old transcripts (Figure 11A). A global analysis of T>C conversion containing transcripts over time, normalized to steady-state abundance (which remained constant in the course of the analysis) for 9430 genes revealed a median mRNA half-life of 4h (Figure 11B). As expected, the half-life of individual transcripts varied by more than one order of magnitude (Figure 11C).

Control over mRNA stability is essential for the temporal order of gene expression. The new method recapitulated the key underlying principles, because regulatory transcripts, associated with GO terms such as 'transcriptional regulation', 'signal transduction', 'cell cycle', or 'development' exhibited significantly shorter half-lives compared to house-keeping transcripts, falling into GO-terms such as 'extracellular matrix', 'metabolic process', or 'protein synthesis' (Figure 11D). In conclusion, the invention enables the accurate assessment of mRNA stability, providing a convenient method to study post-transcriptional gene regulation. As shown, the method recapitulates global post-transcriptional gene regulatory signatures in mouse embryonic stem cells.

### Example 6: Thiol-linked alkylation for the metabolic sequencing of small RNAs

To gain insights into the intracellular kinetics of small RNA silencing pathways, we applied a nucleotide-analog derivatization strategy that bypasses the need for biochemical isolation of labeled RNA species and enables the determination of RNA biogenesis, and turnover kinetics in the context of total RNA (Figure 12): This strategy is based on a well-established 4-thiouridine (s⁴U) metabolic RNA labeling approach but replaces the ineffective and experimentally challenging biotinylation-step with a short chemical treatment that involves the reaction of s⁴U-containing RNA with iodoacetamide, a sulfhydryl-reactive compound, which - upon reaction with s⁴U - creates a covalently linked amidomethylgroup at the base-pairing interface (Figure 1). When combined with conventional RNA library preparation protocols, such as small RNA sequencing, the presence of the bulky group at the sites of s⁴U -incorporation leads to the specific and quantitative misincorporation of G during reverse transcription (RT), but does not interfere with RT-processivity. s⁴U-incorporation events can be identified by sequence comparison, usually bioinformatically in high-throughput sequencing libraries at single nucleotide resolution and in the context of unlabeled RNA by calling T to C transitions, hence eliminates the need for spike-in solutions to measure absolute labeling efficiencies. Importantly, no enzyme that converts uridine to cytosine is known and T>C error rates in high throughput RNA sequencing datasets using e.g. the Illumina HiSeq 2500 platform are rare, occurring at a frequency of less than one in ten thousand. We refer to this approach as thiol(SH)-linked alkylation for the metabolic sequencing of small RNAs.

To test the ability of this method to uncover metabolically labeled small RNAs, we incubated *Drosophila* S2 cells with s⁴U for 24 h under conditions that do not interfere with cell viability (i.e. 500 µM), followed by total RNA extraction and small RNA sequencing. Metabolic labeling was confirmed by HPLC analysis of total RNA (Figure 18), revealing a labeling efficiency of 2.3 %, corresponding to s⁴U incorporation in one out of 43 uridines. Upon generating libraries from size-selected total RNA of *Drosophila* S2 cells after s⁴U metabolic labeling for 24 hours, we observed a strong accumulation of T>C conversions when compared to libraries prepared from unlabeled cells, as exemplified for the miR-184 locus, which gives rise to an abundantly expressed microRNA, miR-184-3p, and its less abundant miR*, miR-184-5p (Figure 12A). In order to confirm this observation at the genomic scale, we aligned reads to annotated microRNA loci in the Drosophila genome and inspected the frequency of any given mutation normalized to overall T-content per miRNA (Figure 12B). In the absence of s⁴U metabolic labeling, we observed a median mutation rate of less than 0.1% for any given mutation, a rate that is consistent with Illumina-reported sequencing error rates. (Note, that iodoacetamide treatment of unlabeled total RNA had no detectable impact on small RNA abundance and mutation rate, as determined by high-throughput sequencing. See Figure 19). After 24 h of s⁴U metabolic labeling, we observed a statistically significant (p<10⁻⁴, Mann-Whitney test), 74-fold increase in T>C mutation rates, while all other mutations remained below expected sequencing error rates (Figure 12B). More specifically, we measured a median s⁴U-incorporation of 2.22% after 24h labeling, corresponding to one s⁴U incorporation in every 45 uridines, and consistent with incorporation rates determined by HPLC measurements of total RNA (Figure 18). Metabolic labeling under unperturbed conditions therefore caused the vast majority (>95%) of small RNAs to exhibit at most one s⁴U incorporation event (Figure 20), which is insufficient for quantitative recovery even through improved biotinylation strategies (Duffy et al., 2015, supra), but can readily be identified by chemical s⁴U derivatization (Figure 12).

The ability of the inventive method to recover s⁴U incorporations at single nucleotide resolution enabled us to systematically dissect the impact of s⁴U metabolic labeling on microRNA processing and loading. To this end, we determined the over- or underrepresentation of T>C conversions at individual positions of a given small RNA that is derived from the 5p- or 3p arm of a microRNA precursor, or that constitutes a miR or miR* strand, as defined by selective Argonaute-loading. When consulting the 71 abundantly expressed (>100 ppm) microRNAs (corresponding to 35 5p- and 36 3p-miRNAs, or 44 miR and 27 miR*) we did not observe a significant systematic alteration in relative T>C mutation rates at any given position (Figure 21). We concluded that s⁴U-metabolic labeling does not impact microRNA biogenesis or loading.

Taken together, s⁴U metabolic RNA labeling in cultured *Drosophila* S2 cells, followed by SLAM-seq quantitatively recovers s⁴U incorporation events in small RNAs at single nucleotide resolution and reveals no significant position-dependent impact of s⁴U labeling on microRNA biogenesis and loading.

### Example 7: Intracellular kinetics of microRNA biogenesis

MicroRNAs are derived from hairpin-containing RNA polymerase II transcript that are sequentially processed by Drosha in the nucleus and Dicer in the cytoplasm, giving rise to mature miRNA duplexes (Figure 13). To investigate the intracellular kinetics of miRNA biogenesis we determined the increase in T>C mutation rates of abundantly expressed miRNAs (>100 ppm at steady-state) in small RNA libraries prepared from size selected total RNA of *Drosophila* S2 cells 5 min, 15 min, 30 min and 60 min after metabolic labeling with s⁴U and compared them to error rates detected in the absence of s⁴U labeling (Figure 13B). Already after a labeling time as short as 5 min, we detected a significant elevation in T>C conversion rates, with 17% of all miRs and 90% of all miR*s exhibiting error rates above the maximum background level. This fraction increases over time with 74%, 93% and 100% miRs after 15 min, 30 min and 1 h, respectively. Based on conservative measures, more than 50% of all miRNAs (22 out of 42) were detectably produced (i.e. exceeding the maximum background T>C conversion rates in either a miR or its respective miR* partner) in a short period of time, i.e. 5 min, revealing a remarkable efficiency in cellular organization of miRNA processing.

We also determined the number of T>C conversion-containing reads as a proxy for the number of miRNA molecules produced over time (Figure 13C). While in average, miRNAs with high steady state abundance also exhibit a high production rate (such as miR-184, or miR-14), others exhibit low steady-state abundance despite high-biogenesis rates (such as miR-276b or miR-190), or vice versa (i.e. miR-980 or miR-11), indicating that the rate at which miRNAs are produced is not the sole determinant for their intracellular abundance.

While our global analysis revealed an unexpectedly high efficiency in overall miRNA biogenesis, selected small RNAs were produced at significantly lower rates. Examples for such ineffectively produced miRNAs were mirtrons (i.e. miR-1003, miR-1006, or miR-1008; Figure 13C), a class of microRNAs that are produced by splicing instead of Drosha-directed processing. Mirtron biogenesis may be selectively dampened, because splicing-derived precursor hairpins are specifically targeted for uridylation by the terminal nucleotidyltransferase Tailor, which recognizes the 3' terminal splice acceptor site, thereby preventing efficient Dicer-mediated processing and triggering dmDis312-directed exonucleolytic decay (Figure 13D). Our data provide experimental evidence for the selective suppression of mirtron biogenesis, because mirtrons exhibit a significantly reduced T>C mutation rates and T>C conversion containing reads accumulated less rapidly over time, when compared to canonical miRNAs (Figure 13D). The hypothesis that uridylation of pre-miRNA hairpins underlies the inhibition of mirtron biogenesis, we also detected a significant correlation between pre-miRNA tailing and T>C conversion rates (Figure 22).

In summary, the new method uncovers a remarkable efficiency in the intracellular rates of miRNA production and recapitulates the selective inhibitory effect of precursor-hairpin uridylation on miRNA biogenesis.

### Example 8: Monitoring small RNA loading into ribonucleoprotein complexes

MicroRNA biogenesis produces miRNA duplexes. But only one of the two strands of a miRNA duplex (the miR strand) is preferentially loaded onto Ago1 and selectively stabilized, whereas the other strand (the miR* strand) is expelled and degraded in the process of miRNA loading (Figure 14A). To test if the inventive method recapitulates the process of miRNA duplex production and miRNA loading in living cells, we analyzed the relative accumulation of T>C conversion containing reads for 20 miRNAs, for which we detected sufficiently high levels of both miR and its partner miR* (Figure 14B). We did not observe a significant difference in abundance between miR and miR* pairs at early timepoints after s⁴U metabolic labeling (i.e. 5 min, 15 min and 30 min; Mann Whitney test p>0.05), confirming that miRNAs initially accumulate as duplexes in a process that is kinetically uncoupled from loading. Only after one hour, we detected a significantly higher accumulation of miR over miR* strands (Mann Whitney test p<0.05, Figure 14B), indicating that - in average - loading of miRNAs into Ago1 occurs at much slower rates compared to miRNA biogenesis.

More detailed analysis uncovered a biphasic process underlying miRNA accumulation: The first phase was identical between miR and miR* (*k*_{miR}=0.35±0.03 and *k*_{miR*}=0.32±0.03), hence reflected the accumulation of miRNAs as a duplex. Notably, a second, slower phase was offset from the biogenesis phase for both miR and miR*. A severe drop in accumulation rates of miR*s (*k*_{miR*}=0.32±0.03) indicated that the vast majority (i.e. ∼81%) of miR* strands undergo rapid degradation as a consequence of miRNA loading. In contrast, miR strands exhibited a much faster second accumulation rate (*k*_{miR}=0.26±0.03) compared to miR* strands (*k*_{miR*}=0.32±0.03), recapitulating the selective stabilization of miR strands, presumably due to miRISC formation. But when compared to the initial biogenesis rate (*k*_{miR}=0.35±0.03), also miRs exhibited a drop in second phase kinetics (*k*_{miR}=0.26±0.03), indicating that only ∼74 % of miR-strands are effectively loaded, while around a quarter of miRNAs are presumably degraded as a duplex. This is consistent with empty Argonaute-availability represents a key limiting factor for the accumulation of miRNAs, and their overexpression globally increases intracellular miRNA abundance.

Further investigation of individual miR:miR* pairs revealed varying loading efficiencies among miRNA duplexes: While strand separation - hence loading -was detectable within minutes in the case of miR-184, *bantam* exhibited slightly delayed loading kinetics with strand separation occurring not before ∼30 min; and miR-282 ranked among the least efficiently loaded miRNAs (Figure 14D). Notably, the different loading kinetics followed thermodynamic rules for Ago1 loading, where mismatches in the seed or 3' supporting region of miRNA duplexes promoted the efficient formation of miRISC.

In summary, the new method revealed detailed insights into miRNA biogenesis and loading kinetics.

### Example 9: IsomiR production

Accumulating evidence suggest that a variety of intracellular processes diversify the sequence and function of microRNAs, but the underlying mechanisms are poorly understood and difficult to dissect from steady-state small RNA sequencing libraries. One well-established example for isomir production is the exonucleolytic maturation of miRNAs in flies. While the majority of miRNAs in *Drosophila* are produced as ∼22 nt small RNAs, selected miRNAs are generated as longer, ∼24mers, which require further exonucleolytic maturation, mediated by the 3'-to-5' exoribonuclease Nibbler to form gene regulatory miRISC. In standard small RNA sequencing libraries and high-resolution Northern hybridization experiments, miR-34-5p exhibits a diverse length profile ranging from abundantly expressed 24-21mer isoforms originating from 3' end truncations of the identical 5' isoform (Figure 15B). To test the ability of the inventive method to disentangle the intracellular order of events that give rise to multiple miR-34-5p isoforms we analyzed small RNA libraries prepared from total RNA of S2 cells after subjecting them to s⁴U-metabolic labeling timecourse. In contrast to steady state small RNAs, which displayed a highly similar length profile across the entire timecourse, T>C conversion containing miR-34-5p reads initially accumulated entirely as a 24mer isoform, consistent with previous in vitro processing experiment employing recombinant Dcr-1 or fly lysate and synthetic pre-miR-34 (Figure 15C, bottom). Only from 3 h onwards, we detected the emergence of shorter, T>C conversion containing miR-34-5p 3' isoforms (Figure 15C top). From this timepoint on, the weighted average length of miR-34-5p over time decreased continuously, slowly approaching the average length profile of miR-34-5p observed at steady-state (Figure 15D). We concluded that the inventive method uncovers the emergence of isomiRs, as exemplified by Nibbler-directed 3'-to-5' exonucleolytic trimming.

Exonucleolytic maturation of miRNAs requires their loading into Ago1 and biochemical evidence suggested that trimming only occurs after miR* strand removal, presumably because Nibbler proposed functions as a single-strand-specific 3'-to-5' exoribonuclease. Because our method enabled us to simultaneously measure miRNA loading and isomiR production, we tested this hypothesis by comparing miR-34-5p trimming signal (Figure 15D) and miR-34 duplex loading kinetics (Figure 15E). We observed that trimming in deed occurred after miR-34 loading and miR* strand removal, as determined by the offset of miR-34-5p and -3p accumulation in our libraries starting after 1h of metabolic labeling (Figure 15E). In conclusion, the inventive method uncovers the intracellular order of miRNA isoform production, hence provides a powerful tool to shed light onto the processes that diversify the sequence and function of miRNAs in living cells.

### Example 10: MicroRNA stability

While different miRNAs assemble into otherwise indistinguishable protein complexes, accumulating evidences suggest that their stability can differ dramatically (Figure 16). But currently available technologies only measure relative, but not absolute half-lifes for individual miRNAs, preventing detailed insights into miRNA stability. Furthermore, metabolic labeling under unperturbed conditions prompts the vast majority (>95%) of labeled small RNAs to exhibit at most one s⁴U incorporation event (Figure 20), which is insufficient for quantitative recovery even through improved biotinylation strategies (Duffy et al., 2015, supra), and introduces biases due to vastly different U-contents in miRNA different miRNA sequences. In contrast, the inventive method provides rapid access to absolute, and sequence content-normalized miRNA stabilities in the context of standard small RNA libraries (Figure 16). By analyzing the T>C conversion rate normalized to miRNA-U-content in SLAM-seq small RNA libraries prepared from total RNA of Drosophila S2 cells subjected to s⁴U metabolic labeling for up to 24 h, we determined a median half-life of 12.13h for the 41 abundantly expressed miR strands (Figure 16B), indicating that the average miRNA half-life is significantly longer compared to mRNAs, which exhibits an average half-life of ∼4-6 hours. In contrast to miRs, miR*s exhibited a much shorter half-life of 0.44 h (Figure 16B), consistent with their enhanced turnover as a consequence of miR-loading (Figure 14). Overall, miR*s are significantly less stable compared to miRs (Figure 16C). But even miRs exhibited intrinsically different stabilities that differed by more than one order of magnitude, as exemplified by the unstable miR-12-5p (t_{1/2}=1.7 h) and the stable *bantam-*5p (t_{1/2}>24 h). Importantly, the inventive method provides robust insights into individual small RNA half-lifes, providing highly reproducible results across a wide variety of small RNA stabilities (Figure 16E).

MicroRNA stability is a major contributing factor to the establishment of small RNA profiles in S2 cells, as exemplified by the two miRNAs that accumulated to highest levels at steady-state: While *bantam* exhibited relatively slow biogenesis (Figure 13) and medium loading rates (Figure 14), it accumulated to highest steady-state levels because of an unusually high stability (t_{1/2}>24 h). In contrast, the second most abundant miRNA, miR-184-3p, was 3-times less stable compared to bantam-5p (t_{1/2}=6 h), but still accumulated to high levels because of its extraordinarily high biogenesis and loading kinetics (Figure 13 and 14). The metabolic sequencing of small RNAs by SLAM-seq therefore uncovers the relative contribution of miRNA biogenesis, loading and turnover to the establishment of steady-state small RNA profiles, the major determinant for miRNA-mediated gene regulation.

### Example 11: Argonaute protein identity defines small RNA stability

The genomes of both mammals and insects, encode several proteins of the Argonaute protein family, some of which selectively load small RNAs to regulate distinct subsets of transcripts by varying mechanisms. While miRNA duplexes are intrinsically asymmetric, i.e. the miR strand preferentially loads into Ago1, each miRNA precursor can potentially produce two mature small RNA strands that are diffentially sorted into two distinct ubiquitously expressed Argonaute proteins in flies. In contrast to the majority of miRs, miR*s are often loaded as functional species into Ago2, the effector protein in the RNAi pathway, and undergo selective methylation at the 2' position of the 3' terminal ribose by the methyltransferase Hen1 in the final step of Ago2-RISC assembly (Figure 17A). Depletion of Ago2 does not compromise cell viability, and enabled us to investigate the role of Ago proteins in the selective stabilization of small RNAs. Furthermore, it provided an experimental framework to understand if small RNA half-lifes are intrinsically determined by the identity of the Ago protein they associate with.

We first established the set of miRNAs that specifically assembled into Ago2 in wild-type *Drosophila* S2 cells by comparing small RNA libraries generated from total RNA by conventional small RNA cloning (predominantly reflecting Ago1-bound small RNAs) to libraries generated from total RNA but enriching for methylated (i.e. Ago2-bound) small RNAs by oxidation. While the majority of small RNAs in the conventional cloning approach consisted of miRNAs (particularly miR strands), oxidation selectively enriched for Ago2-bound endogenous small RNAs derived from transposons, genes (predominantly derived from overlapping mRNA transcripts) and loci giving rise to long fold-back transcripts (structured loci). As described previously, the subset of methylated (i.e. Ago2-bound) miRNAs was selectively enriched for miR*s. Comparison of unoxidized and oxidized small RNA libraries enabled us to classify miR and miR* strands according to their accumulation in Ago1 or Ago2 (Figure 17C). By comparing the abundance of Ago2-enriched small RNAs in conventional small RNA libraries generated from wild-type S2 and S2 cells depleted of Ago2 by CRISPR/Cas9 genome engineering (Figure 17D), we confirmed that Ago2-enriched small RNAs are significantly less abundant upon depletion of Ago2 (p<0.002, Wilcoxon matched-pairs signed rank test, Figure 17E).

We next determined the stability of Ago2-enriched small RNAs by measuring small RNA stabilities in total small RNA libraries prepared from wild-type and Ago2-depleted (*ago2*^{ko}) cells by s⁴U metabolic-labeling followed by SLAM-seq. In wild-type cells, Ago2-enriched small RNAs followed a two-phase decay kinetic, where the majority (i.e. 94%) of the population exhibited high stability (t_{1/2}>24 h) and only a minority did undergo rapid decay, with a half-life similar to miR*s (t_{1/2}=0.2 h). We tested if the population associated with long half-life might represent the Ago2-bound fraction by determining the stability of the same small RNA species in methylated small RNA libraries. Indeed, Ago2-enriched small RNAs followed single-exponential decay kinetics with a half-life of >24 h (Figure 17F). Conversely, in Ago2-depleted S2 cells, Ago2-enriched small RNAs again followed dual-phase decay kinetics, but now the majority (63%) of the population exhibited miR*-like stability (t_{1/2}=0.4 h), indicating that in the absence of Ago2, these small RNAs are predominantly decayed upon loading of their partner-strand into Ago1. In contrast, Ago1-enriched small RNAs had identical stabilities in the presence and absence of Ago2 (Figure 17F). Our data therefore unravels population-specific stabilities of miRNAs that are determined by their loading into specific Ago-proteins. Finally, to dissect if small RNA half-lifes are intrinsically determined by the identity of the Ago protein they associate with we compared the stabilities of the 30 most abundant small RNAs in Ago1 and Ago2 (Figure 17G). This analysis revealed that Ago2-bound small RNAs exhibited a significantly higher stability compared to Ago1-bound small RNAs (p<10⁻⁴; Mann Whitney test), perhaps because methylation of Ago2-bound small RNAs contributes to the stabilization of small RNAs in Ago2 but not Ago1.

In summary, we provide an experimental framework for the dissection of the molecular mechanisms underlying the establishment and maintenance of small RNA profiles that impact gene expression states in health and disease.

## Claims

1. Method of identifying a polynucleic acid (PNA) comprising the steps of providing a PNA; modifying one or more nucleobases of the PNA by addition or removal of a hydrogen bonding partner, thereby altering the base pairing capacity of the one or more nucleobases; base pairing a complementary nucleic acid to the PNA, including base pairing to at least one modified nucleobase; identifying the sequence of the complementary nucleic acid at least at the position that is complementary to at least one modified nucleobase.

2. The method according to claim 1, wherein the step of modification is by inclusion of a thiol modified nucleobase and alkylating said thiol nucleobase with an alkylating agent that comprises the hydrogen bonding partner.

3. The method according to claim 1, wherein the modification comprises alkylating on position 4 of a uridine with an alkylating agent that comprises the hydrogen bonding partner.

4. The method according to any one of claims 1 to 3, wherein the PNA comprises one or more 4-thiouridine.

5. The method according to any one of claims 1 to 4, wherein a thiol modified nucleobase is incorporated into the PNA through biosynthesis in a cell, preferably wherein modifying one or more nucleobases comprises attaching or removing a hydrogen bonding partner to the thiol modified nucleobase.

6. The method of any one of claims 1 to 5, wherein base pairing to at least one modified nucleobase leads to base paring with another nucleotide than base pairing with a nucleobase that has not been modified, with said nucleobases being otherwise the same.

7. The method according to any one of claims 1 to 6, wherein the PNA comprises or consists of RNA or DNA.

8. The method according to any one of claims 1 to 7, wherein for each nucleotide type selected from A, G, C, U or T the modified PNA comprises more natural nucleotides than modified nucleotides.

9. The method according to any one of claims 1 to 8, wherein the PNA comprises 1, 2, 3, 4, 5, 6, 7, 8, 9, 10 or more and up to 30 modified nucleotides.

10. The method of any one of claims 1 to 9, wherein providing a PNA comprises expressing the PNA in cell; said method further comprises isolating the PNA from the cell; modifying one or more nucleobases of the PNA in the cell and/or after isolation; wherein the modification(s) in the cell or after the isolation or both together add or remove a hydrogen bonding partner of one or more nucleobase, thereby altering the base pairing capacity of the one or more nucleobases.

11. The method according to any one of claims 1 to 10, wherein one or more cells are cultured or grown in at least two culturing or growth phases, wherein one culturing or growth phase comprises incorporation of a modified nucleotide into biosynthesized PNA, which is modified by addition or removal of a hydrogen bonding partner, and another culturing or growth phase that lacks such incorporation of the modified nucleotide into biosynthesized PNA or wherein modified nucleotides are incorporated into biosynthesized PNA at a different concentration as in the other one culturing or growth phase; or wherein the method comprises incorporation of a modified nucleotide into biosynthesized PNA of at least two different cells or into at least two different groups of cells, wherein preferably the incorporation of the two different cells or two different groups of cells is compared.

12. The method according to claim 11, wherein the biosynthesized PNA of the two culturing or growth phases or of the of at least two different cells or at least two different groups of cells are collected from said cells, preferably also mixed, especially preferred with labelling the PNA according to the cell origin of the PNA, and wherein base pairing a complementary nucleic acid to the PNA comprises generation of complementary polynucleic acid strands, preferably DNA strands, by transcription, preferably reverse transcription.

13. The method of claim 12, further comprising determining the sequence of the complementary polynucleic acid strands and comparing the strand sequences, wherein an altered complementary nucleic acid as a result of the modification by addition or removal of a hydrogen bonding partner can be identified by comparison with the complementary nucleic acid without modification.

14. A kit for performing a method of any one of claims 1 to 13 comprising a thiol modified nucleobase and an alkylating agent suitable for alkylating the thiol modified nucleobase at the thiol group, wherein the alkylating agent comprises a hydrogen boding donor or acceptor, preferably wherein the alkylating agent is iodoacetaminde.

15. The kit of claim 14 further comprising primers, nucleotides selected from A, G, C, and T, a reverse transcriptase or a combination thereof, preferably all these components.
